# EUROPÄISCHE PATENTSCHRIFT

(11) **EP 2 289 090 B1**
(45) Veröffentlichungstag und Bekanntmachung des Hinweises auf die Patenterteilung: **28.07.2021**
(21) Anmeldenummer: 09745541.4
(22) Anmeldetag: 28.04.2009
(51) Int. Cl.: H01J 49/00, G06F 1/00, G01N 24/00, G16B 30/00

(54) **VERFAHREN UND ANORDNUNG ZUR STEUERUNG VON MESSSYSTEMEN, SOWIE EIN ENTSPRECHENDES COMPUTERPROGRAMM UND EIN ENTSPRECHENDES COMPUTERLESBARES SPEICHERMEDIUM**
METHOD AND ARRANGEMENT FOR THE CONTROL OF MEASURING SYSTEMS, CORRESPONDING COMPUTER PROGRAMME AND CORRESPONDING COMPUTER-READABLE STORAGE MEDIUM
PROCÉDÉ ET DISPOSITIF DE COMMANDE DE SYSTÈMES DE MESURE, PROGRAMME D'ORDINATEUR CORRESPONDANT, ET SUPPORT D'ENREGISTREMENT LISIBLE PAR ORDINATEUR CORRESPONDANT

(30) Priorität: 28.04.2008 DE 102008021703
(43) Veröffentlichungstag der Anmeldung: 02.03.2011
(73) Patentinhaber: Thermo Fisher Scientific (Bremen) GmbH, 28199 Bremen (DE)
(72) Erfinder: Kühn, Andreas, 28209 Bremen (DE); Lindscheid, Michael W., 12203 Berlin (DE); Tham, Katja, 12459 Berlin (DE); Freytag, Johann-Christoph, 14532 Kleinmachnow (DE); Heymann, Stephan, 10435 Berlin (DE)
(74) Vertreter: Stellbrink & Partner Patentanwälte mbB
(86) Internationale Anmeldenummer: PCT/EP2009/003355
(87) Internationale Veröffentlichungsnummer: WO 2009/138207

(56) Entgegenhaltungen:
- WO-A1-96/36986
- US-A1- 2005 063 864
- US-A1- 2006 043 281
- US-A1- 2006 192 101
- US-A1- 2007 187 588
- P. H. Dawson ET AL: "The use of triple quadrupoles for sequential mass spectrometry: 2-A detailed case study", OMS. ORGANIC MASS SPECTROMETRY., vol. 17, no. 5, 1 May 1982 (1982-05-01), pages 212-219, XP055571307, GB ISSN: 0030-493X, DOI: 10.1002/oms.1210170504
- J. Mitchell Wells ET AL: "Collision-Induced Dissociation (CID) of Peptides and Proteins" In: "METHODS IN ENZYMOLOGY", 1 January 2005 (2005-01-01), ACADEMIC PRESS, US, XP055571323, ISSN: 0076-6879 vol. 402, pages 148-185, DOI: 10.1016/S0076-6879(05)02005-7,
- Ivan Haller ET AL: "Collision induced decomposition of peptides. Choice of collision parameters", JOURNAL OF THE AMERICAN SOCIETY FOR MASS SPECTROMETRY., vol. 7, no. 7, 1 July 1996 (1996-07-01), pages 677-681, XP055571351, US ISSN: 1044-0305, DOI: 10.1016/1044-0305(96)85613-3
- "Introduction to mass spectrometry, Chapter 3: Mass Spectrometry / Mass Spectrometry ED - Watson J Throck; Sparkman Orrin David", 1 January 2007 (2007-01-01), INTRODUCTION TO MASS SPECTROMETRY : INSTRUMENTATION, APPLICATIONS, AND STRATEGIES FOR DATA INTERPRETATION, WILEY, CHICHESTER [U.A], PAGE(S) 173 - 228, XP002729973, ISBN: 978-0-470-51634-8

## Beschreibung

Die Erfindung betrifft ein Verfahren und eine Anordnung zur Steuerung von Messsystemen, sowie ein entsprechendes Computerprogramm und ein entsprechendes computerlesbares Speichermedium, welche insbesondere einsetzbar sind, um Messgeräte automatisch durch zielorientiertes Eingreifen in ein Messexperiment zu steuern, wobei die Steuerung auf einer Online Datenanalyse der jeweils aktuellen Messungen basiert. Je nach Messexperiment kann dabei eine serielle oder parallele Messstrategie verfolgt werden, bei der das kombinierte Ergebnis der Datenanalyse entweder einen direkten Einfluss auf die nächste Messung hat oder in einer dynamisch organisierten Sequenz an Messungen resultiert. Die Art, in der bestimmte Geräteparameter abhängig von der Aussage verändert werden, wird für ein Messexperiment durch die Variationsstrategie festgelegt. Bei den Messexperimenten kann es sich insbesondere um Aufklärung von Monomersequenzen von Biopolymeren mittels MS (Mass Spectrometry), um Aufklärung der 3D-Molekularstruktur mittels NMR (NuclearMagnetic Resonance) o.dgl. handeln.

Analyten sind analysierbare, messbare Stoffe die durch verschiedenartige Messgeräte hinsichtlich ihrer Eigenschaften und ihrem Verhalten untersucht werden können. Eigenschaften und Verhalten von Analyten wird nachfolgend gemeinsam unter dem Begriff Eigenschaften verstanden.

Anhand von Messdaten aus Messexperimenten, die eine bestimmte Messanordnung und Durchführung besitzen, können Ergebnisse abgeleitet und im Sinne der verwendeten Auswertungsstrategie kombiniert und als Aussagen über Analyten von Interesse interpretiert werden.

Kommt es in Messanordnungen zu einer zeitabhängigen Änderung von Analyten oder Analytzusammensetzungen, so ergeben sich Zeitfenster, in denen bestimmte Messdaten zugänglich sind. Diese Annordnungen werden im Folgenden als zeitkritische Messexperimente bezeichnet.

Die Menge aller ableitbaren, im Sinne der Auswertungsstrategie kombinierbaren Ergebnisse eines Messexperiments wird im Folgenden als Gesamtaussage bezeichnet und zur Beantwortung einer gegebenen Fragestellung (Definition einer Fragestellung folgt im weiteren Textteil) herangezogen. Gesucht wird die Menge an ableitbaren, kombinierten Einzelergebnissen (Aussagen), die sich zu einer Gesamtaussage verknüpfen lassen und welche die Fragestellung eindeutig und vollständig beantwortet.

Zur Automatisierung der Messexperimente werden derzeit Methoden verwendet, die durch starre Messabfolgen charakterisiert sind. Jede Messung basiert auf einem festgelegten Satz an Geräteparametern, der den Messmodus des Gerätes vorgibt.

Diese Messmodi können entweder vorab definiert oder auf Grundlage von aktuellen Messdaten und resultierenden Datenbezügen für den weiteren Messverlauf bestimmt werden. Die Einflussnahme in den Messverlauf ist jedoch nur sehr beschränkt möglich, da bisher einzig Messdaten und direkt aus der Messung ableitbare Datenbezüge zwischen diesen als Indikatoren verwendet werden , um vordefinierte Messungen auszulösen. Diese Indikatoren allein sind jedoch unzureichend, um Aussagen bezüglich komplexerer Fragestellung zu extrahieren.

Eine Auswertung wird bislang in einem, dem Messlauf nachgeschalteten Prozessierungsschritt durchgeführt. Kann die vorgegebene Fragestellung nicht mit der hierbei ableitbaren und im Sinne der gewählten Auswertungsstrategie kombinierbaren Ergebnissen (Gesamtaussage) beantwortet werden, sind weitere Messexperimente mit ggf. veränderten Messmodi notwendig. Dieses Prinzip resultiert in einer seriellen Abfolge von Messläufen und Prozessierungsschritten bis eine Beantwortung möglich ist oder keine weiteren Messungen mehr durchführbar sind. Dieser Prozess kann mitunter sehr zeitaufwendig sein, da eine stets nachgelagerte Auswertung die zielorientierte Änderung bestimmter Messmodi erst nach Abschluss eines gesamten Experimentes zulässt. Es entsteht somit ein erhöhter Verbrauch an messtechnischen Ressourcen und einer möglicherweise begrenzten Menge an zu untersuchenden Analyten. Zudem entstehen große Mengen an redundanten Daten.

In der Patentanmeldung US 2007/0187588 wird ein Verfahren zur Analyse von Proben wie Proteinen beschreiben, bei dem gemessene Massenspektren dissoziierter Massen (MS² Spektren) mit in einer Datenbank gespeicherten entsprechenden Massenspektren von Referenzproben verglichen werden. Dieser Vergleich beruht auf der Prüfung, ob in beiden Spektren die Peaks gleicher Komponenten bei entsprechend gleichen m/z-Werten vorhanden sind und ob die Peaks gleicher Komponenten die gleiche Intensität aufweisen. Wird durch diese Prüfung ein Unterschied der Massenspektren festgestellt, wird eine weitere Messung an der untersuchten Probe im nicht dissoziierten Zustand durchgeführt.

In der Patentanmeldung WO 96/36986 A1 wird ein Verfahren und eine Vorrichtung zum Sequenzieren von Polymeren unter Verwendung von Massenspektrometrie offenbart. Im Verfahren wird ein Polymer mit unterschiedlichen Mengen Hydrolysemittel in verschiedenem Umfang fragmentiert und mit einem Massenspektrometer analysiert. Das Verfahren umfasst ferner die Analyse der Daten, die aus der Massenspektrometrieanalyse der Vielzahl von Reihen hydrolysierter Polymerfragmente erhalten wurden und stellt statistische Interpretationsmöglichkeiten und Computersoftware dafür bereit.

Die Aufgabe der vorliegenden Erfindung besteht somit darin, ein Verfahren und eine Anordnung zur Steuerung von Messsystemen, sowie ein entsprechendes Computerprogramm und ein entsprechendes computerlesbares Speichermedium bereitzustellen, welche die Nachteile der bekannten Lösungen vermeiden und es insbesondere ermöglichen, komplexe Bezüge zwischen Messdaten auszuwerten und auf Basis der abgeleiteten Auswertungsergebnisse und/oder daraus abgeleiteter, interpretierter Aussagen während des Ablaufs eines Messexperiments zielgerichtet in das Messexperiments einzugreifen.

Die beschriebene Erfindung ist vorzugsweise in den Fällen einsetzbar, in denen folgende Kriterien/Bedingungen erfüllen sind.
I. Das verwendete Messsystem besitzt Geräteparameter, die über ein Steuermodul variiert werden können und deren jeweiliger Einfluss auf die Messung definiert ist bzw. werden kann. Zudem besitzt das Messsystem ein Messdaten-Modul zur Ausgabe der gewonnenen Messwerte einer Messung.
II. Eine betrachtete Fragestellung ist prinzipiell mit einem Messexperiment auf dem verwendeten Messsystem zu beantworten bzw. eindeutig lösbar. Mögliche Fragestellungen sind bspw. a) die Aufklärung von Eigenschaften und Verhalten der betrachteten Messobjekte anhand der auftretenden Messwerten, b) die Aufklärung von Einflüssen von Geräteparametern und Kombinationen von diesen auf Messdaten und resultierenden Bezüge, c) das Auffinden von Geräteparametern und/oder Geräteparametersätzen, bei denen eine bestimmte Menge an Messdaten und/oder eine bestimmte Menge an Datenbezügen auftreten und d) eine beliebige Kombination von a)-c).
III. Zur Lösung der Fragestellung oder Teilfragestellungen existiert jeweils mindestens eine Auswertungsstrategie, welche die zur Lösung benötigten Aussagen, wie bspw. Informationen von Eigenschaften und dem Verhalten von Messobjekten und die Vorgehensweise, diese aus Teilaussagen, wie bspw. Informationen über Teileigenschaften zu kombinieren definiert. Sind alle benötigten Teilaussagen bzw. Informationen zu Teileigenschaften eindeutig und vollständig zu ermitteln, so ist auch die Lösung der Fragestellung bzw Teilfragestellung vollständig und eindeutig.
IV. Alle theoretisch auftretenden Messdaten und alle daraus konstruierbaren Datenbezüge der zu untersuchenden Messobjekte, lassen sich rechnerisch und kombinatorisch generieren und mit jeweils daraus ableitbaren Aussagen, wie bspw. Informationen zu (Teil-)Eigenschaften der betrachteten Messobjekte, verknüpfen. Dabei können auch zusätzliche (nicht vom Messsystem generierbare) Informationen mit eingebunden werden, wobei die zugrundeliegenden Datenbezüge fast beliebig komplex und fast beliebig umfangreich sein können. Der für die Fragestellung relevante Teil von Aussagen bzw. Informationen bildet, verknüpft mit den entsprechenden/ zugrundeliegenden Messdaten und Datenbezügen die Datenbasis, welche vor dem Messexperiment erzeugt wird.
V. Einfache Datenanfragen von Messdaten und Datenbezügen an diese Datenbasis erzeugen die benötigten Aussagen, wie bspw. Informationen zu Teileigenschaften und ggf. damit verbundene Aussagen. Der resultierende Geschwindigkeitsvorteil gegenüber dem zuvor verwendeten rechnerisch-kombinatorischen Ansatz (Erzeugung) ermöglicht dabei das Ermitteln von Aussagen bzw. Informationen zu Eigenschaften der zu untersuchenden Messobjekte, die ansonsten zur Laufzeit (zeitnah) nicht zugänglich sind.
VI. Direkt nach der Auswertung einer Messung lässt sich bestimmen ob eine Aussage, wie bspw. die Information über eine Eigenschaft eindeutig und vollständig ermittelt werden konnte oder welche Teilaussagen bzw. Teileigenschaften nur uneindeutig oder unvollständig ermittelt werden konnten.
VII. Die zu der selben Fragestellung zugehörigen Aussagen, bspw. die zur selben Eigenschaft eines bestimmten Messobjekts zugehörigen Informationen über Teileigenschaften können aus unterschiedlichen Messungen/Auswertungen zusammengefasst werden, um eine Vollständigkeit und Eindeutigkeit zu gelangen.
VIII. Für ein Messexperiment wird eine messsystem-spezifische Variationsstrategie festgelegt, die abhängig von der Fragestellung entweder
   für a) bei uneindeutigen und unvollständigen bestimmbaren Informationen Teileigenschaften aus der Auswertung einer Messung, die Geräteparameter dahin gehend verändert, so dass "damit gerechnet werden kann", dass a) bei unvollständigen Informationen über Teileigenschaften, die noch fehlenden erlangt werden können und b) bei uneindeutigen Ermittlung, die fehlenden Informationen erlangt werden können, die über die zugrundeliegenden Teileigenschaften benötigt werden.
   für b) bestimmt, wie ein betrachteter Geräteparameter oder einen Geräteparametersatz variiert wird, um die relative Veränderung aller auftretenden Messdaten und Datenbezüge zu erfassen.
   für c) bestimmt wie ein betrachteter Geräteparameter oder den Geräteparametersatz systematisch variiert, um somit die jeweils resultierenden Messdaten mit der gesuchten Menge an Messdaten/Datenbezügen zu vergleichen.
IX. Für die Durchführung eines Messexperimentes wird eine Messstrategie festgelegt, die bestimmt, in welcher Vorgehensweise die oben genannten Variationsstrategien (der Geräteparameter) angewandt werden. Je nach gewünschtem oder bedingtem Fokus, kann die Variation
   a) in direkter Form stattfinden. Die Geräteparameter, der nächsten Messung werden aufgrund der aktuellen Messung festgelegt. Die Mess- und Auswertungsabfolge ist dann seriell (Messung-Auswertung-Messung-Auswertung), b) in indirekter/verzögerter Form. Jede Auswertung bedingt den Eintrag einer Messung mit veränderten Geräteparametern in einer (Aufgaben) Messliste, die abgearbeitet wird. Die Mess- und Auswertungsabfolge ist dann parallel.

Diese Aufgabe wird erfindungsgemäß gelöst durch die Merkmale der Ansprüche 1, 19, 20, 21, 22. Zweckmäßige Ausgestaltungen der Erfindung sind in den Unteransprüchen enthalten.

Ein besonderer Vorteil des erfindungsgemäßen Verfahrens besteht darin, dass zeitkritische Messexperimente, wie beispielsweise die Aufklärung von Aminosäuresequenzen in Biopolymeren mittels Massenspektrometrie, während der Laufzeit des Messexperimentes auf Basis von Messdaten beeinflusst werden können, welche während dieses Messexperiments gewonnen wurden. Dies wird erfindungsgemäß dadurch erreicht, dass eine Datenbasis bereitgestellt wird. Die Datenbasis umfasst Informationen über Beziehungen zwischen zumindest einem Teil der Messdaten. Die Datenbasis umfasst zumindest einen Teil der bei der Durchführung eines Messexperiments gewinnbaren Messdaten und/oder zumindest einen Teil von aus den gewinnbaren Messdaten herleitbaren Informationen. Bei diesen Informationen kann es sich vorzugsweise um Beziehungen zwischen gewinnbaren Messdaten handeln, wie beispielsweise Differenzen zwischen Paaren von Messdaten. Vorzugsweise werden in der Datenbasis Daten abgespeichert, welche einen Großteil des Ergebnisraums, vorzugsweise den gesamten Ergebnisraum, der bei dem Messexperiment erfassbaren Messdaten darstellen. Darüber hinaus kann die Datenbasis weitere Informationen über chemisch und/oder physikalische Eigenschaften der Messobjekte sowie über Beziehungen zwischen Messwerten (Datenbezüge) umfassen, die bei einer Anfrage an die Datenbasis ebenfalls ausgewertet werden können.

Eine bevorzugte Ausführungsform der Erfindung sieht vor, dass es sich bei dem Messexperiment um die Aufklärung von Aminosäuresequenzen von Biopolymeren, insbesondere von Peptiten/Proteinen, mittels Massenspektrometrie (MS) oder um die Aufklärung von 3D-Molekularstrukturen mittels Kernmagnetresonanz (NMR) handelt, in diesem Falle kann es sich bei den zu vermessenden Messobjekten um Biopolymere und/oder deren Derivate und/oder bei den Messdaten um Massenangabe von Aminosäurenoder Aminosäuresequenzen, um Masse-/Ladung-Verhältnisse und/oder die Intensität von Signalen handeln. Mehrere miteinander verkettete Monomere bilden ein Polymer, und mehrere Monomere und/oder Polymere bilden ein Polymer oder ein Derivat eines Polymers oder Untereinheiten davon. Unter Monomeren, Biopolymeren oder Derivaten werden im folgenden auch modifizierte Monomere, modifizierte Biopolymere bzw. modifizierte Derivate verstanden. Insbesondere kann es sich bei den Biopolymeren oder Derivaten um Proteine, Peptide, DNA, RNA, PNA, LNA, TNA, GNA, Polysaccharide, wie Stärke, Cellulose oder Glykogen, um Lipide, Polyglucosamine, wie Chitin oder Chitosan, um Polyhydroxyalkanoate, Cutin, Suberin oder Lignin oder Kombinationen davon handeln.

Bei den Informationen über Beziehungen zwischen gewinnbaren Messdaten kann es sich beispielsweise um Differenzen zwischen Messdaten, insbesondere um Massendifferenzen, handeln. In einer bevorzugten Ausführungsform der Erfindung umfasst die Datenbasis Massendifferenzen zwischen jeweils zwei Monomeren und/oder Polymeren der Biopolymere. Vorzugsweise umfasst die Datenbasis auch Massendifferenzen zwischen Polymeren, wobei sich die Polymere um mehrere Monomere unterscheiden.

Die Datenbasis umfasst Informationen über zumindest einen Teil der zu vermessenden Messobjekte. Neben den Angaben über erzielbare Messdaten oder Beziehungen zwischen den Messdaten kann die bereitgestellte Datenbasis weiter Informationen über bereits durchgeführte Messexperimente umfassen.

Erfindungsgemäß werden während des Ablaufs des Messexperiments die aktuell erfassten Messdaten automatisch ausgewertet, wobei die Auswertung die Ermittlung von Beziehungen zwischen zumindest einem Teil der erfassten Messdaten umfasst. Auf Basis der ermittelten Beziehungen erfolgt anschließend eine Anfrage an die Datenbasis, um Informationen über erfasste Messobjekte zu erhalten. Dabei dienen die ermittelten Beziehungen wenigstens zum Teil als Anfrageparameter. In einer bevorzugten Ausführungsform der Erfindung erfolgt eine Anfrage, wobei zumindest ein Teil der Massen von Biopolymerfragmenten eines Fragmentspektrums und zumindest ein Teil der Massendifferenzen zwischen den Biopolymerfragmenten als Anfrageparameter dienen. Durch die Anfrage werden mögliche Kandidaten für unaufgeklärte Sequenzteile der Biopolymere ermittelt, die bei einer massenspektrometrischen Fragmentierung entstehen.

In der Datenbasis sind Informationen über Beziehungen zwischen den Messdaten mit weiteren Informationen verknüpft, welche Angaben über zu vermessende Messobjekte umfassen. In Abhängigkeit einer Auswertestrategie für das Messexperiment und in Abhängigkeit der Informationen über erfasste Messobjekte, welche durch die Anfrageparameter identifiziert werden, erfolgt nun eine Steuerung des Messsystems.

In einer bevorzugten Ausführungsform der Erfindung ist vorgesehen, dass die Datenbezüge in Form einer Tabelle oder einer, auch mehrdimensionalen, Matrix aufgebaut ist. Die Tabellenbeziehungsweise Matrixeinträge entsprechen in einer bevorzugten Ausführungsform den ermittelten Beziehungen zwischen Messdaten, wie beispielsweise Massendifferenzen, und die Zeilen beziehungsweise Spalten beziehen sich auf Messobjekte. Eine bevorzugte Ausführungsform sieht dabei vor, dass Elemente einer Matrix jeweils einer Massendifferenz entsprechen, welche denjenigen Polymeren beziehungsweise Polymerfragmenten zugeordnet sind, deren Massen sich um diese Massendifferenz unterscheiden, und die beispielsweise den Zeilen bzw. Spalten zugeordnet sind, in der die Massendifferenz eingetragen ist. Die Massen dieser (Bio-)Polymere beziehungsweise (Bio-)Polymerfragmente würden dann einander zugeordnet. Im Ergebnis der Anfrage werden Zuordnungsinformationen generiert, die beispielsweise Angaben darüber umfassen, welche (Bio-)Polymerfragmente sich um die Massendifferenz unterscheiden. Die in der Matrix aufgefundene Massendifferenz würde dann der Differenz der Massen der beiden zugeordneten (Bio-)Polymerfragmente entsprechen. Darüber hinaus können die Zuordnungsinformationen Angaben zu dem Monomere oder dem Polymerfragment umfassen, durch welche sich die beiden zugeordneten (Bio- )Polymerfragmente unterscheiden.

In einer bevorzugten Ausführungsform der Erfindung werden diese Zuordnungen verwendet, um mit Hilfe von Verfahren der Graphentheorie, Verfahren der künstlichen Intelligenz, wie z.B. Fuzzylogik, (neural network, genetische algo und/oder evolutionsstrategien..) mögliche Kandidaten für unaufgeklärte Sequenzteile zu ermitteln. In einer bevorzugten Ausführungsform der Erfindung wird dafür ein ungerichteter Graph erstellt, wobei für jede durch die Anfrage ermittelte Masse in Knoten erzeugt wird. Zwischen Knoten, das heißt Massen, welche einer Massendifferenz der Anfrage zugeordnet wurden, wird eine Kante definiert.

In einer bevorzugten Ausführungsform der Erfindung werden Graphentheoretische Ansätze verwendet um mögliche Kandidaten für unaufgeklärte Sequenzteile zu ermitteln. Es werden entlang der Kanten von einer Masse ausgehend in Richtung ansteigender bzw. abfallender Massen Verknüpfungsketten erzeugt (Pfade). Vorzugsweise beginnt man bei der Bildung der Verknüpfungsketten bei dem Knoten mit der niedrigsten Masse. Dann werden alle Verbindungsketten ermittelt, die entlang der Kanten zu Knoten jeweils höherer Masse verlaufen bis der Knoten mit der höchsten Masse erreicht ist. Die Bildung der Verknüpfungsketten kann natürlich auch ausgehend von dem Knoten mit der höchsten Masse in absteigender Richtung bis hin zum Knoten mit der geringsten Masse erfolgen. Die Knoten innerhalb einer Verbindungskette werden im folgenden auch als Elemente, die Kanten innerhalb einer Verbindungskette als Verknüpfungen bezeichnet. Den Kanten werden vorzugsweise Informationen über ein oder mehrere Monomere zugeordnet, welche die Massendifferenz zwischen den beiden durch die Kante verknüpften Knoten (Massen) repräsentieren. Mögliche Kandidaten für unaufgeklärte Sequenzteile des Biopolymers werden dann dadurch angezeigt, wenn zwei Massen durch eine Kante verbunden sind, die sich um eine Massendifferenz von mehr als einem Monomer unterscheiden.

Das Vorhandensein unaufgeklärter Sequenzteile wird somit dadurch angezeigt, dass Verknüpfungen (Kanten) zwischen Elementen (Knoten) der Verknüpfungskette existieren, denen Massendifferenzen zugeordnet sind, die mindestens der Massendifferenz von zwei Monomeren entsprechen, oder wenn die niedrigste Masse nicht der Masse eines einzelnen Monomers entspricht, oder die Massendifferenz der höchsten Masse zum unfragmentierten Biopolymer genau zwei Monomeren entspricht. Umgekehrt wird die vollständige Aufklärung der Sequenz eines Biopolymers durch eine Verknüpfungskette angezeigt, deren Verknüpfungen (Kanten) ausschließlich die Masse eines einzelnen Polymers repräsentieren.

Je nach den verfügbaren Geräteparametern, (bspw.Fragmentierungsmethoden und entsprechende Parametern) und den zu erwartenden Einflüssen sämtlicher Parameter, wird für die möglichen Aussagen der Fragestellung (bspw. unvollständiger/uneindeutiger Sequenzabdeckung) eine Variationsstrategie festgelegt. Wird bspw. ein unaufgeklärter Sequenzteil ermittelt, so ist definiert, welche (Einzel)aussagen dazu beigetragen haben. Es kann nun a) ein Fragment (mit entsprechender Masse) ausgewählt werden, das diesen unaufgeklärten Sequenzteil enthält. Bei mehren Kandidaten wird der gewählt, der eine ausreichend hohe Intensität besitzt um für eine weitere Fragmentierung verwendet zu werden und möglichst wenig bekannte Sequenzteile enthält. Zudem können b) ein oder mehrere Parameter verändert werden, so dass es bspw. zu einer stärkeren Fragmentierung kommen sollte (Fachwissen über Methode oder Evaluierung durch DBnovo) oder c) eine andere Fragmentierungsmethode gewählt werden, die komplementäre/zusätzliche Informationen liefern sollte (bspw. Seitenketten Fragmentierung) (Fachwissen über Methode oder Evaluierung durch DBnovo).

Ist es notwendig oder von Vorteil, die sich direkt anschließende Messung von der Aussage der aktuellen Messung abhängig zu machen, so wird eine serielle Messstrategie verfolgt. Hier werden Messung und entsprechende Auswertung im ständigen Wechsel betrieben. Eine ableitbare Aussage kann dann zeitnah zu einer Entscheidung/Eingriff in den Messverlauf führen. Ggf. kann sich der Zeitbdarf der Auswertung nachteilig auf die mögliche Messdichte auwirken.

Ist man darauf aus eine hohe Messdichte zu erlangen, so wird eine parallele Messstrategie verfolgt, d.h. das Gerät führt eine Messung nach der andern aus, ohne auswertungsbedingte Verzögerungen. Die Auswertungen werden parallel zu den Messungen durchgeführt und resultieren durch ihre Aussage und der festgelegten Variationsstrategie zu neuen Messungen. Diese Messungen werden dabei in einer Messliste dynamisch verwaltet. Die Messliste wird nach und nach abgearbeitet und kann bspw. durch Abschätzung der Verfügbarkeitsdauern, durch vollständigen Aufklärung oder durch andere Priorisierungskriterien von Messobjekten in ihrer Reihenfolge und Umfang verändert werden. Hierbei können zeitkritische Messexperimente teilweise sogar zeiteffizienter organisiert und kontrolliert werden.

Eine Anordnung nach der Erfindung weist mindestens einen Chip und/oder Prozessor auf, wobei die Anordnung derart eingerichtet ist, dass ein Verfahren zur Steuerung von Messsystemen ausführbar ist, wobei eine Datenbasis bereitgestellt wird, welche Informationen über zumindest einen Teil der zu vermessenden Messobjekte, über zumindest einen Teil der Messdaten, die bei mit dem Messsystem durchgeführten Messexperimenten erfassbar sind, und Informationen über Beziehungen zwischen zumindest einem Teil der Messdaten umfasst. Während des zeitlichen Verlaufs eines Messexperiments
- werden erfasste Messdaten automatisch ausgewertet, wobei die Auswertung eine Ermittlung von Beziehungen zwischen zumindest einem Teil der erfassten Messdaten umfasst;
- erfolgt mindestens eine Anfrage an die Datenbasis, um mit Hilfe der ermittelten Beziehungen Informationen über erfasste Messobjekte zu erhalten; und
- wird in Abhängigkeit der Informationen über erfasste Messobjekte das Messsystem gesteuert.

In der bevorzugten Ausführungsform aus dem Bereich der Massenspektrometrie handelt es sich bei dem Messsystem um ein Massenspektrometer. Eine Datenbasis umfasst in diesem Falle vorzugsweise unter anderem Massendifferenzen zwischen Monomeren und/oder Polymeren der Biopolymere, insbesondere auch Massendifferenzen zwischen Polymeren, die sich um ein oder mehrere Monomere unterscheiden. Bei der Anfrage an die Datenbasis können Kandidaten für noch nicht aufgeklärte Sequenzteile der Biopolymere ermittelt werden, wobei diese beispielsweise durch Massendifferenzen von mehreren Monomeren angezeigt werden. Basierend auf solchen ermittelten, noch nicht aufgeklärten Sequenzteilen können dann die Geräteparameter, wie beispielsweise zugeführte Energiepulse, gesteuert werden.

Ein Computerprogramm zur Messgerätesteuerung ermöglicht es einer Datenverarbeitungseinrichtung, nachdem es in den Speicher der Datenverarbeitungseinrichtung geladen worden ist, ein Verfahren zur Steuerung von Messsystemen durchzuführen, wobei eine Datenbasis bereitgestellt wird, umfassend Informationen über zumindest einen Teil der zu vermessenden Messobjekte, über zumindest einen Teil der Messdaten, die bei mit dem Messsystem durchgeführten Messexperimenten erfassbar sind, und umfassend Informationen über Beziehungen zwischen zumindest einem Teil der Messdaten;
während des zeitlichen Verlaufs eines Messexperiments
- erfasste Messdaten automatisch ausgewertet werden, wobei die Auswertung eine Ermittlung von Beziehungen zwischen zumindest einem Teil der erfassten Messdaten umfasst;
- mindestens eine Anfrage an die Datenbasis erfolgt, um mit Hilfe der ermittelten Beziehungen Informationen über erfasste Messobjekte zu erhalten; und
- in Abhängigkeit der Informationen über erfasste Messobjekte das Messsystem gesteuert wird.

In einer weiteren bevorzugten Ausführungsform der Erfindung ist vorgesehen, dass das erfindungsgemäße Computerprogramm modular aufgebaut ist, wobei einzelne Module auf verschiedenen Datenverarbeitungseinrichtungen installiert sind.

Vorteilhafte Ausführungsformen sehen zusätzlich Computerprogramme vor, durch welche weitere in der Beschreibung angegebene Verfahrensschritte oder Verfahrensabläufe ausgeführt werden können.

Solche Computerprogramme können beispielsweise (gegen Gebühr oder unentgeltlich, frei zugänglich oder passwortgeschützt) downloadbar in einem Daten- oder Kommunikationsnetz bereitgestellt werden. Die so bereitgestellten Computerprogramme können dann durch ein Verfahren nutzbar gemacht werden, bei dem ein Computerprogramm nach Anspruch 29 aus einem elektronischen Datennetz, wie beispielsweise aus dem Internet, auf eine an das Datennetz angeschlossene Datenverarbeitungseinrichtung heruntergeladen wird.

Um das erfindungsgemäße Verfahren zur Steuerung von Messsystemen durchzuführen, ist vorgesehen, ein computerlesbares Speichermedium einzusetzen, auf dem ein Programm gespeichert ist, das es einer Datenverarbeitungseinrichtung ermöglicht, nachdem es in den Speicher der Datenverarbeitungseinrichtung geladen worden ist, ein Verfahren zur Steuerung von Messsystemen durchzuführen, wobei eine Datenbasis bereitgestellt wird, umfassend Informationen über zumindest einen Teil der zu vermessenden Messobjekte, über zumindest einen Teil der Messdaten, die bei mit dem Messsystem durchgeführten Messexperimenten erfassbar sind, und umfassend Informationen über Beziehungen zwischen zumindest einem Teil der Messdaten;
während des zeitlichen Verlaufs eines Messexperiments
- erfasste Messdaten automatisch ausgewertet werden, wobei die Auswertung eine Ermittlung von Beziehungen zwischen zumindest einem Teil der erfassten Messdaten umfasst;
- mindestens eine Anfrage an die Datenbasis erfolgt, um mit Hilfe der ermittelten Beziehungen Informationen über erfasste Messobjekte zu erhalten; und
- in Abhängigkeit der Informationen über erfasste Messobjekte das Messsystem gesteuert wird.

Ein besonderer Vorteil der vorliegenden Erfindung besteht in einer schnellen Auswertung von Massenspektren hinsichtlich einer gesetzten Fragestellung mit der Möglichkeit, den aktuellen

Messverlauf zielführend zu verändern. Dies erschließt einen großen Anwendungsbereich in der Massenspektrometrie.
- Es ist möglich Untersuchungsreihen zu Fragmentierungsmechanismen durchzuführen, bei denen mehrere Fragmentierungsparameter für bestimmte Aminosäure-Abfolgen nach einer Systematik durchvariiert werden. Die resultierenden Veränderungen sind direkt zur Laufzeit ableitbar und auswertbar. Es können gezielt Aussagen verfolgt und dokumentiert werden.
- Geräteparameter können für bestimmte Aminosäure-Abfolgen anhand von resultierenden Massenspektren optimiert werden.
- Neuartige Fragmentierungsreaktionen und -methoden können für den Einsatz gezielter, regiospezifischer Bindungsspaltungen optimiert werden. Dies könnte für eine sukzessive Fragmentierung von großen Biomolekülen an verschiedenen Bindungsstellen (Aminosäureabfolgen) genutzt werden, um durch die resultierenden überlappenden Fragmente eine vollständige Sequenzierung zu erhalten.

Die Erfindung wird nachfolgend unter Bezugnahme auf die Figuren der Zeichnungen an einem Ausführungsbeispiel näher erläutert. Es zeigen:
Figur 1 Skizzierte Darstellung einer automatisierten und zielorientierten Messgerätesteuerung basierend auf einer datenbankgestützten Online-Datenanalyse und Echtzeit-Rückkopplung resultierender Messergebnisse,
Figur 2 Beispiel der Aufklärung von Aminosäuresequenzen in Biopolymeren:
   Fragment der verschiedenen Serien (a, b, c-Serie (mit N-Terminus) und x, y, z-Serie (mit C-Terminus)) am Beispiel des Pentapeptids RQKGK,
Figur 3 Beispiel der Aufklärung von Aminosäuresequenzen in Biopolymeren:
   Zeitlicher Ionen-Intensitätsverlauf einer Menge von Peptiden im Massenspektrometer (MS) bei vorgeschaltetem Trennsystem (HPLC),
Figur 4 Beispiel der Aufklärung von Aminosäuresequenzen in Biopolymeren:
   Veranschaulichung einer starren Messabfolge: MS-Übersichtsscan (A) und nachfolgende Fragmentierung der drei intensivsten Peaks mittels IRMPD: MS2(IRMPD,464.25) (B), MS2(IRMPD,653.36) (C), MS2(IRMPD,585.02) (D),
Figur 5 Beispiel der Aufklärung von Aminosäuresequenzen in Biopolymeren:
   Veranschaulichung der Ermittlung von Massen aus Messdaten bei einer Auswertung von Fragmentspektren,
Figur 6 Zum Beispiel der Aufklärung von Aminosäuresequenzen in Biopolymeren: Veranschaulichung der Erzeugung einer Matrix von Massendifferenzen,
Figur 7a Zum Beispiel der Aufklärung von Aminosäuresequenzen in Biopolymeren: Veranschaulichung der Erstellung eines Graphen aus Ergebnissen einer Messanfrage,
Figur 7b Beispiel der Aufklärung von Aminosäuresequenzen in Biopolymeren:
   Abstrahierte Veranschaulichung des DBnovo Auswertungsverfahrens zur Erstellung von Aussagen aus den Messdaten einer Messung,
Figur 8 Zum Beispiel der Aufklärung von Aminosäuresequenzen in Biopolymeren: Schematische Veranschaulichung einer beispielhaften Ermittlung von Pfaden in einem Graphen,
Figur 9a Beispiel der Aufklärung von Aminosäuresequenzen in Biopolymeren:
   Extrahierte Ionenspuren von drei Peptiden aus einem HPLC/MS Messexperiment,
Figuren 9b-d Beispiel der Aufklärung von Aminosäuresequenzen in Biopolymeren:
   Die Erhöhung der Sequenzabdeckung nach veränderten Messmodi ist für jedes Peptid aufgeführt. Insgesamt werden 10 Messungen benötigt, um die drei Peptide eindeutig zu identifizieren. Die Differenz zu den bisher notwendigen Messungen (45 Scans) kann für die Identifizierung weiterer, in diesem Zeitfenster auftretender Peptide genutzt werden.

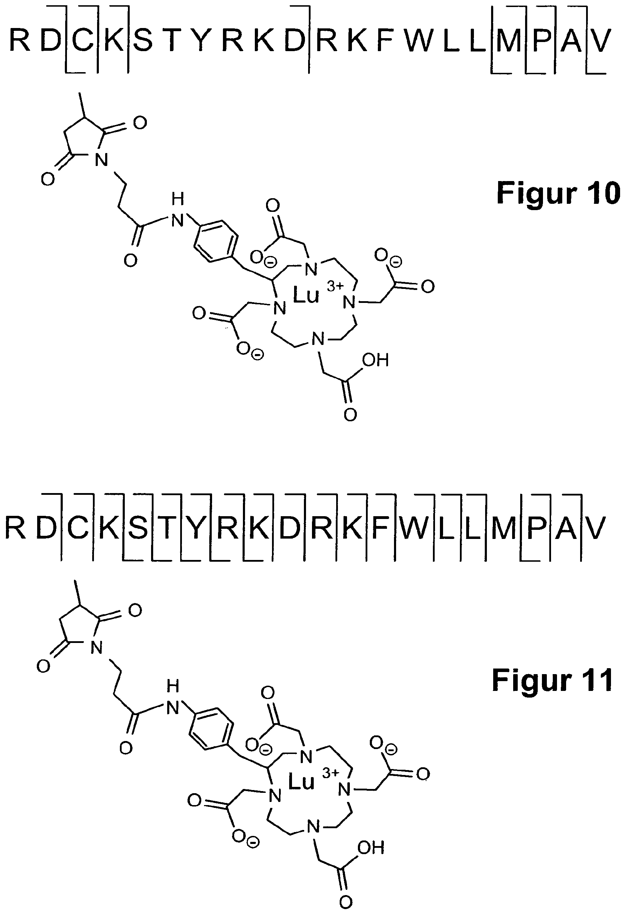

Figur 10 Beispiel der Aufklärung von Aminosäuresequenzen in Biopolymeren: Veranschaulichung einer Auswertung eines Fragmentspektrums bei Verwendung eines ersten Fragmentierungsmodus und
Figur 11 Beispiel der Aufklärung von Aminosäuresequenzen in Biopolymeren: Veranschaulichung einer Auswertung eines Fragmentspektrums bei Verwendung eines zweiten Fragmentierungsmodus.

In folgenden soll die Erfinder näher am Beispiel der Aufklärung von Aminosäuresequenzen in Biopolymeren erläutert werden. Die Erfindung ist natürlich nicht auf diese spezielle beispielhafte Ausführungsform eingeschränkt, sondern umfasst ebenso andere Messvorgänge, wie z.B. Strukturaufklärungen wie etwa eine Aufklärung der 3D-Molekularstruktur oder dgl., solange nur die in den unabhängigen Ansprüchen definierten Merkmale realisiert werden.

### Kurze abstrahierte Beschreibung der Erfindung:

Die hier beschriebene Erfindung umfasst eine automatisierte Steuerung von Messgeräten, die auf der Online Datenanalyse der jeweils aktuellen Messungen basiert, um in das Messexperiment zielorientiert einzugreifen.

Bisherige Ansätze verwenden einzig Messdaten und direkt aus der Messung ableitbare Datenbezüge zwischen diesen als Indikatoren, um vordefinierte Messungen auszulösen. Diese Indikatoren allein sind jedoch unzureichend, um aus ableitbaren und im Sinn der Auswertungsstrategie verknüpfte Ergebnissen, Aussagen bezüglich komplexerer Fragestellung zu extrahieren. Hierzu sind neben zusätzlichen Bezügen zu bereits bekannten Analytinformationen ebenso Bezüge, die auf Grundlage einfacher und/oder auf Basis anderer Bezüge gebildet wurden notwendig.

In einem Messexperiment wird die jeweils aktuelle Messung derart ausgewertet, dass (Teil)Fragestellungen durch - aus den Messdaten ableitbare Aussagen (in Form interpretierter und im Sinne der Auswertungsstrategie kombinierter Ergebnisse) - beantwortet werden können. Die Ableitung dieser Aussagen erfordert je nach Art der Fragestellung und verwendetem Messmodus eine bestimmte Auswertungsstrategie, die mitunter sehr komplex gestaltet sein kann und auf der Erzeugung einer unterschiedlich hohen Anzahl an Datenbezügen basiert. Um Aussagen zur Laufzeit zu erhalten und in den Messverlauf einzugreifen, solange die Messung noch aktuell ist, d.h. bevor eine Änderung der Analyten oder Analytzusammensetzung eintritt, wird ein schnelles Verfahren zur Auswertung der Datenbezüge benötigt.

Je nach anfallenden Datenmengen und bestehender Auswertungskomplexität, kann ein rechnerischer Algorithmus sehr zeitaufwendig und besonders bei zeitkritischen Messexperimenten ungeeignet sein. Lassen sich für ein Messexperiment jedoch alle auftretenden Möglichkeiten, d.h. alle konstruierbaren Datenbezüge aus allen theoretischen Messwerten der zu untersuchenden Analyten und/oder Analytzusammensetzung rechnerisch und kombinatorisch generieren, so ist der vollständige Ergebnisraum der Auswertung bzw. ein für das Messexperiment ausgewählter Teil davon durch eine Datenbasis darstellbar. Diese verknüpft dabei einzelne Datenbezüge des erzeugten Ergebnisraums mit Informationen über Messobjekte, deren Eigenschaften und/oder Datenbezügen innerhalb dieser. Die erzeugten Datenbezüge jeder Messung, sind somit durch einfache Datenanfragen an eine vor dem Experiment generierbaren Datenbasis auswertbar. (Gilt für eine Menge an Messexperimenten/ bestimmte Messanordnung)

Die hier vorgestellte Erfindung bedient sich der Geschwindigkeit solcher Datenanfragen, und ermöglicht dadurch eine Echtzeit-Rückkopplung der abgeleiteten Aussagen auf den nachfolgenden Messverlauf. Dies führt zu einer automatisierbaren und auch hinsichtlich komplexer (Teil)Fragestellungen zielorientierten Einflussnahme in die Messsteuerung zur Laufzeit.

Die vorliegende Erfindung zur automatisierten und zielorientierten Messgerätesteuerung ist insbesondere, jedoch nicht ausschließlich, für folgende Szenarien mit entsprechenden Fragestellungen und Zielen anwendbar:
- Szenario 1:
   Beantwortung von Fragestellungen im Bezug auf Analyteigenschaften Beispiel: Die Aufklärung von Aminosäuresequenzen von Peptiden/Proteinen mittels MS (Mass Spectrometry)
   Die Effizienz von Messexperimenten kann hinsichtlich der Vollständigkeit und Eindeutigkeit der Aminosäuresequenzen von Peptiden/Proteinen gesteigert werden, indem nach jeder Messung eine Aussage über Struktur-/Sequenzaufklärung vorliegt und anhand ungeklärter Struktur-/Sequenzteile ein gezieltes Anpassen der Geräteparameter - zur Laufzeit - vollzogen wird.
   Gegenüber bisherigen Ansätzen können entweder die Anzahl benötigter Messungen für die Aufklärung der Aminosäuresequenzen deutlich reduziert werden bzw. der Informationsgehalt der Sequenzinformation deutlich gesteigert werden.
- Szenario 2:
   Aufklärung des Einflusses/wechselseitigen Abhängigkeiten einzelner oder gekoppelter Geräteparameter auf das resultierende Messergebnis Wie ändert sich bspw. das Fragmentierungsverhalten einer Gruppe von Biopolymeren, wenn die Zeit zwischen zwei zugeführten Energieimpulen verändert wird?
   Durch Detektion aller möglichen Fragmentteile und die einhergehender Intensitätsänderungen dieser Fragmente mit Änderung der Geräteparameter, kann eine ggf. auftretende Regioselektivität und Sequenzspezifität festgestellt werden. Eine Erfoschung, was eine Veränderung des Geräteparameters bewirkt ist möglich. Auch die Unterscheidung, ob ein Effekt mit Erhöhung oder Erniedrigung der Parameters verstärkt oder abgeschwächt wird ist ermittelbar. Dies kann bei der Variation des Parameters zur Evaluierung genutzt werden, um somit Bereiche, in denen kein Effekt mehr zu verzeichnen ist zu vernachlässigen. Es ist somit das "Tunen" von Parametern auch bei dem Einsatz mehrerer Parameter möglich. Neue Geräteoptionen/Messmethoden, können somit für bestimmte Anwendungen einsetzbar gemacht und für eine Grundfunktionalität optimiert werden. Ergebnisse aus dieser Anwendungsmöglichkeit können zur Definition von Standardparameter oder von Parametern für spezifische Fälle genutzt werden.
- Szenario 3:
   Die Optimierung von Geräteparameter(-sätzen), um ein definiertes Messergebnis zu erhalten
   Beispiel: Ein bestimmter Geräteparametersatz wird für ein Instrument gesucht, der zur Spaltung eines Analyten an einer gewünschten/vorgegebenen Position führt.
   Ähnlich wie in Szenario 2 können Geräteparameter und Sätze an Geräteparametern variiert werden, um deren Einfluss auf die Messung bspw. der Fragmentierungseffizienz zu ermitteln. Sind die Einflüsse der eingesetzten Geräteparameter nur schwer einschätzbar, besonders, wenn die Anzahl an Kombinationsmöglichkeiten zwischen gleichzeitig eingesetzten Geräteparametern der Fragmentierungsmethoden hoch ist, so kann man sie für einen gewünschten Einfluß optimieren lassen. Sucht man die optimalen Parametersätze , um einen favorisierten Bindungsbruch zu erlangen, so kann durch systematische Variation analysiert werden, wann die hierfür spezifischen Fragmente vermehrt auftreten, ohne dass unerwünschte Fragmente entstehen. Auch kann eine Einschätzung erfolgen, in welche Richtungen die Variation der Parameter getrieben werden sollte, um sich diesem Ziel zu nähern. Ergebnisse aus dieser Anwendungsmöglichkeit können zur Definition von Standardparameter oder von Parametern für spezifische Fälle genutzt werden.

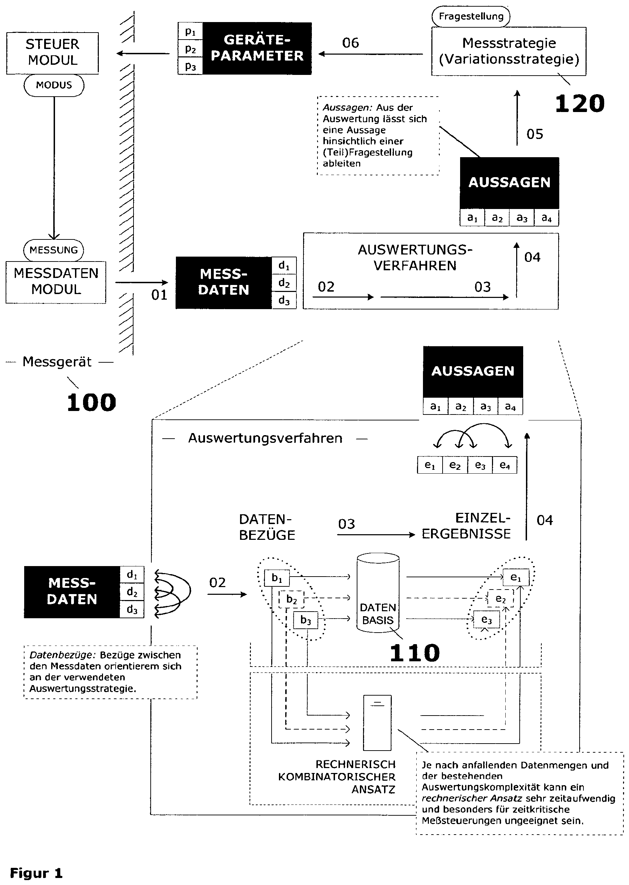

Anhand von Figur 1 soll nachfolgend eine beispielhafte automatisierte, zielgerichtete Steuerung eines Messgeräts 100 beschrieben werden.

In Schritt 01 erfolgt das Aufbereiten der Messwerte und die Dekonvolution dieser. Schritt 01 umfasst insbesondere das Aufbereiten der Messwerte aus der aktuellen Messung hin zu verarbeitbaren Messdaten d₁, d₂, d₃. Dieser Schritt kann in Teilen bereits vom Messgerät 100 übernommen werden.

Schritt 02 umfasst das Erzeugen der Datenbezüge. Es werden dabei Bezüge zwischen den entstandenen Messdaten d₁, d₂, d₃ erzeugt, resultierend in Datenbezügen b₁, b₂, b₃. Die Art der Datenbezüge b₁, b₂, b₃ wird durch die gewählte Auswertungsstrategie bestimmt. In einer beispielhaften Ausführungsform aus dem Bereich der Massenspektrometrie handelt es sich bei den Messdaten d₁, d₂, d₃ um Massenwerte von Biopolymeren, hier Aminosäuren oder Aminosäuresequenzen, die zueinander in Beziehung gesetzt werden, indem die Differenzen zwischen zumindest einem Teil der Massenwerte gebildet werden.

Schritt 03, das Auswerten der Datenbezüge b₁, b₂, b₃, dient dem Auswerten der Datenbezüge b₁, b₂, b₃ unter Zuhilfenahme von unmittelbar ausgeführten Online-Datenanfragen an eine vorliegende bzw. vorberechnete Datenbasis 110.

In Schritt 04 werden Aussagen a₁,a₂,a₃ abgeleitet. Es werden dabei die Einzelergebnisse e₁, e₂, e₃ derart abgeleitet und kombiniert, dass Aussagen a₁, a₂, a₃ zur Beantwortung von (Teil)Fragestellungen getroffen werden können. Die Folgerung dieser Aussagen a₁, a₂, a₃ erfordert je nach Art der Fragestellung eine bestimmte Auswertungsstrategie und stützt sich auf die vorab erzeugten Datenbezüge b₁, b₂, b₃ und deren Verknüpfungen innerhalb der Datenbasis. Bei der erwähnten beispielhaften Massenspektrometrie kann eine Fragestellung etwa die noch nicht aufgeklärten Sequenzbereiche betreffen. Werden solche nicht aufgeklärten Sequenzbereiche festgestellt, etwa indem Massendifferenzen ermittelt werden, die auf eine Sequenz aus mindestens zwei Aminosäuren hinweist, kann eine aus den kombinierten Einzelergebnissen abgeleitete Aussage a₁, a₂, a₃ beispielsweise in der Angabe dieser nicht aufgeklärten Sequenzbereiche bestehen.

In Schritt 05 werden die Aussagen a₁,a₂,a₃ mit der zu erreichenden Fragestellung abgeglichen. Aus der Auswertung lässt sich eine abgeleitete Aussage a₁, a₂, a₃ hinsichtlich einer (Teil)Fragestellung ableiten. Schritt 05 wird genutzt, um einen Abgleich der extrahierten Aussagen a₁, a₂, a₃ mit der Menge der festgelegten Teilfragestellungen (> Ziel) vorzunehmen, mit dem Ziel im Folgenden Strategien zum weiteren Messverlauf bzw. zur Variation der Geräteparameter abzuleiten.

In Schritt 06 erfolgt eine zielorientierte Variation der Geräteparameter p₁, p₂, p₃, d.h. die aus den aktuellen Messwerten extrahierten abgeleiteten Aussagen a₁, a₂, a₃ fließen direkt (durch eine im wesentlichen Echtzeit-Rückkopplung) in die folgenden Messungen des Experimentes ein.
(siehe allgemein: Variationsstrategie)
Anwendungsbereich: Massenspektrometrie

Datenbankgestützte Online De-Novo Sequenzierung von Biopolymeren
Eine mögliche Anwendung der hier beschriebenen Erfindung besteht in der datenbankgestützte Online-Identifizierung von Biopolymeren (bspw. Peptide, Proteine, DNA, RNA) mittels Massenspektrometrie und zielorientierter Steuerung des Massenspektrometers.

In der Proteomforschung wird die Massenspektrometrie eingesetzt, um Primärstrukturen von Proteinen und Peptiden, d.h. ihre Aminosäuresequenzen und ggf. auftretende Modifikationen aufzuklären (Identifizierung).

Eingesetzte Massenspektrometer detektieren dabei Masse/Ladungs-Verhältnisse der untersuchten Biopolymere, die in ionisierten Formen oder kurz als Ionen vorliegen. Jede Messung resultiert in einem Massenspektrum, welches das Masse/Ladungs-Verhältnis der Intensität gegenüberstellt. Die entstehenden gaussförmigen Verteilungen der den Ionen zugehörigen Messdaten werden im Folgenden als Peaks bezeichnet. Aus den Abständen zwischen den Peaks lassen sich Ladungen bestimmen, aus welchen sich die Massen (m) der Peptide errechnen (Dekonvolution).

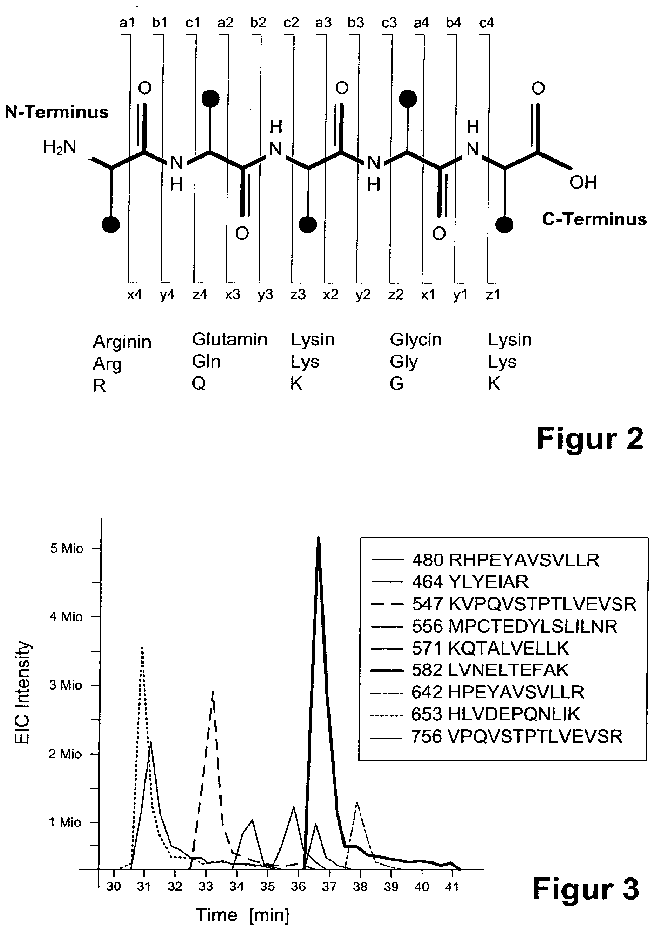

Nachdem alle Peptidmassen in einem Übersichts-Massenspektrums (MS) ermittelt wurden, werden im nächsten Schritt Peptidionen selektiert und zur Strukturaufklärung in kleinere Bruchstücke fragmentiert (Tandem-Massenspektrometrie, MSn). Es entstehen Fragmentspektren, in denen abhängig von der verwendeten Fragmentierungsmethode (CID = Electron Capture Dissociation oder Elektronenemfangs Dissoziation, IRMPD = Infrared Multiphoton Dissociation oder Infrarot Multiphotonen Dissoziation, ECD = Collition Induced Dissociation oder Kollisionsinduzierte Dissoziation) spezifische Fragmente des ausgewählten Vorläuferions (Peptid) beobachten werden können. Diese können in verschiedene Serien eingeteilt werden (a,b,c-Serie bzw. x,y,z-Serie). Die verschiedenen Serien sind in Figur 2 am Beispiel des Pentapeptids RQKGK zusammengefasst dargestellt und bilden sich formal durch eine sukzessive und serienspezifische Abspaltung der einzelnen Aminosäuren ausgehend von einem Ende des Peptids (C/N-Terminus). Abhängig von der Fragmentierungseffizienz und damit bedingt durch Parameter wie der normalisierten Kollisionsenergie (bei CID), der Laserleistung (bei IRMPD) oder der Dauer und Verzögerung der Einwirkungen von Elektronen (ECD), kommt es zu einer unterschiedlich hohen Abdeckung der beobachtbaren Fragmentserien. Je nach Vollständigkeit und Überlappungen der Fragmentserien kann die vollständige und eindeutige Abfolge der Aminosäuren bestimmt werden.

Der Messaufbau zur Identifizierung von Peptidgemischen beinhaltet im Normalfall ein Trennsystem wie bspw. High Performance Liquid Chrommatigraphy (HPLC), welches direkt an ein Massenspektrometer gekoppelt ist (z.B. LTQ-FT). Dies führt dazu, dass die verschiedenen Peptide in unterschiedlichen Zeitfenstern in das Massenspektrometer gelangen und analysiert werden können (Figur 3). Abhängig von der Auftrennung können die Zeitfenster, in denen das Peptid verfügbar ist, stark variieren und sich zudem teilweise stark überschneiden, was zu so genannten zeitkritischen Messungen führt. Daraus folgt, dass für jedes Peptid ggf. nur wenige Messungen zur Verfügung stehen, um diese eindeutig zu identifizieren. Dies und der Umstand, dass auch die vorherrschenden Konzentrationen der Peptide stark variieren können, machen es bei einem Messexperiment unabdingbar, zur Laufzeit zu entscheiden, wann welche Messungen für ein Peptid durchgeführt werden soll.

Derartige Entscheidungen können jedoch nur auf der Grundlage ausgewerteter Massenspektren getroffen werden, da sich nur aus diesen Aussagen hinsichtlich der Vollständigkeit und Eindeutigkeit der zum jeweiligen Peptid gehörigen Aminosäuresequenz ableiten lassen.

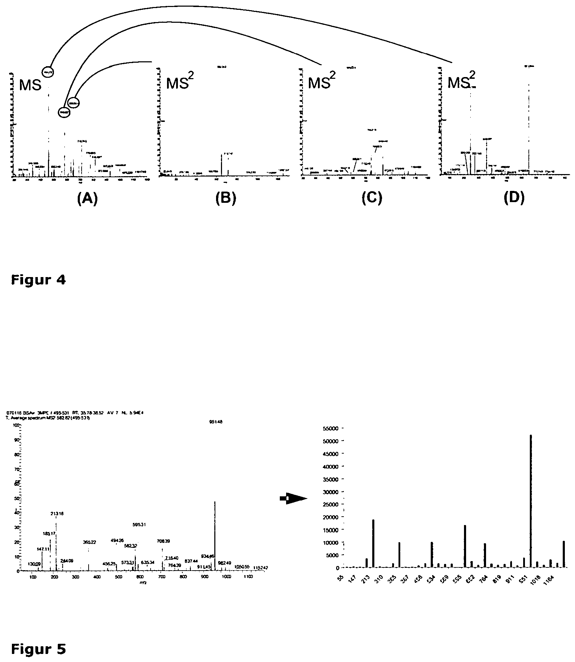

Die Steuerung des Messverlaufs eines Massenspektrometers übernehmen bisher vordefinierbare Messmethoden, die nur beschränkt dynamisch arbeiten und im Normalfall einen starren Wechsel zwischen den vordefinierten Messmodi (bspw. MS, MSn) vornehmen. Im Folgenden ist beispielhaft das Schema einer verbreiteten Vorgehensmethode angegeben und in Figur 4 anhand von Messenspektren visualisiert:
**Messmodus 1.**
   Aufnahme eines Übersichts-Spektrums mit allen auftretenden Ionen (MS)
**Messmodus 2.**
   Fragmentierung des 1. intensivsten Ionen-Signals aus Übersichtsspektrum (1) (MS2)
**Messmodus 3.**
   Fragmentierung des 2. intensivsten Ionen-Signals aus Übersichtsspektrum (1) (MS2)
**Messmodus 4.**
   Fragmentierung des 3., intensivsten Ionen-Signals aus Übersichtsspektrum (1) (MS2) ... gehe zu Messmodus 1.

Die in Figur 4 auszugsweise dargestellte Messabfolge wird unabhängig von der Fragmentierungseffizienz oder extrahierbaren Aussagen der einzelnen Spektren über den gesamten Trennlauf beibehalten. Eine solche Vorgehensweise führt zwangsläufig zu redundanten und für eine Sequenzierung unnötigen Daten und vernachlässigt zudem gering konzentrierte Peptide niedriger Intensitäten. Die zusätzliche Nutzung vordefinierbarer Listen mit bevorzugten und auszuschließenden Ionenmassen, sowie Exklusionslisten, die während des Messexperiments bereits fragmentierte Ionen von weiteren Messungen ausschließen, können lediglich die Menge an möglichen Vorläuferionen eingrenzen.

Ohne Informationen über die bereits vorliegenden Messungen (Messdaten d₁, d₂, d₃) der Peptide und dem damit verbundenen Rückschluss (Aussagen a₁, a₂, a₃) auf Vollständigkeit und Eindeutigkeit der untersuchten Aminosäuresequenzen, kann zur Laufzeit nicht optimal auf das Auftreten verschiedener Ionen reagiert werden.

Anhand der Massendifferenz (Δm) zwischen zwei Peptidfragmenten eines Fragmentspektrums kann die Menge an möglichen Aminosäuren (inklusive deren Modifikationen) ausgemacht werden, in denen sie sich unterscheiden.

Die Strategie, eine Mengen von Massendifferenzen (Δm) zu verwenden, um die Fragmentserien eines Peptids aufzudecken, wird als De-novo Sequenzierung bezeichnet und setzt die Kenntnis der Massen aller Aminosäuren und mit einzubeziehender modifizierten Formen voraus. Sind die beobachteten Fragmentserien des untersuchten Peptids vollständig oder überlappen diese ausreichend stark, so kann die Sequenz eines Peptids durch das sukzessive Anreihen von Fragmenten, die sich jeweils nur um eine Aminosäure (ggf. inklusive auftretender Modifikation) unterscheiden, bestimmt werden (vgl. Figur 2).

Eine Schwierigkeit bei De-novo Sequenzierung besteht darin, alle möglichen Kombinationen von Aminosäuren und ihren modifizierten Formen, in die Bestimmung des Massenabstands einzubeziehen. Der rechnerische Aufwand steigt mit der Anzahl der möglichen Modifikationen so stark an (exponentiell), dass eine Anwendung zur Laufzeit besonders bei zeitkritischen Messungen nicht möglich ist.

Die hier beschriebene Erfindung löst dieses Problem, indem die notwendige Datenbasis 110 mit allen möglichen Kombinationen an Aminosäuren, inklusive ihren möglichen Modifikationen als Masse-Sequenz-Paar vorab erzeugt werden. Die auftretenden Massendifferenzen (Δm) können somit durch einfache Datenanfragen den möglichen Aminosäureformen zugeordnet werden.

Dieses neuartige Verfahren erlaubt es, die Messwerte der Fragmentspektren durch den Geschwindigkeitsvorteil von Datenanfragen sofort dahingehend auszuwerten, wie vollständig ein ausgewähltes Ion (bspw. ein Peptid) durch die vorhandenen Messdaten identifizierbar ist. Wir bezeichnen dieses Verfahren als Online De-novo Sequenzierung. Ausgehend von der Kenntnis über bereits erreichte Teilidentifizierungen, können die Messparameter (Geräteparameter p₁, p₂, p₃) des Massenspektrometers für den weiteren Messverlauf bewusst variiert werden. Diese Rückkopplung der erhaltenen Informationen auf den laufenden Messprozess ermöglicht besonders bei zeitkritischen Messungen (bspw. bei HPLC/MS) einen zielorientierten Eingriff in den Messprozess, während die zu untersuchenden Ionen noch verfügbar sind.

Das für diese beispielhafte Anwendung gewählte Massenspektrometer (LTQ-FT) erzeugt in gepulsten Zyklen Massenspektren nach einem jeweils vorgegebenen Messmodus. Die Zyklendauer beträgt im Mittel etwa ∼1s. Dieses Zeitfenster sehen wir als Richtmaß für die Auswertung eines Massenspektrums und Rückkopplung der erhaltenen Ergebnisse auf die kommenden Messungen an.

Im Folgenden wird die detaillierte Verfahrensweise (als Abfolge der Einzelschritte) - welche wir nachfolgend als DBnovo bezeichnen - für die Auswertung von Fragmentspektren aufgeführt und mit der in verschiedenen Versuchen ermittelten zeitliche Ausdehnung der Teilprozesse zum Zweck der Einordnung/Klärung der Machbarkeit belegt.

### Schritt 01: Aufbereiten der Messwerte und Dekonvolution

Nach Dekonvolution und Filtern der Messdaten d₁, d₂, d₃ (Peaks), werden die Massen der auftretenden Fragmente ermittelt und zur weiteren Prozessierung bereitgestellt (vgl. Figur 5). Hierbei können die durchschnittliche Anzahl der Signale pro Messung, die Ermittlung von Signalen mit gleichem Informationsgehalt, der Schwellwert für Signalrauschen (d.h. bis zu welchem Wert gilt ein Signal als nutzbar, bzw. wird dem allgemeinen Rauschen der Messung zugeschrieben) o.dgl. variieren.

### Schritt 02: Erzeugen der Datenbezüge b1, b2, b3

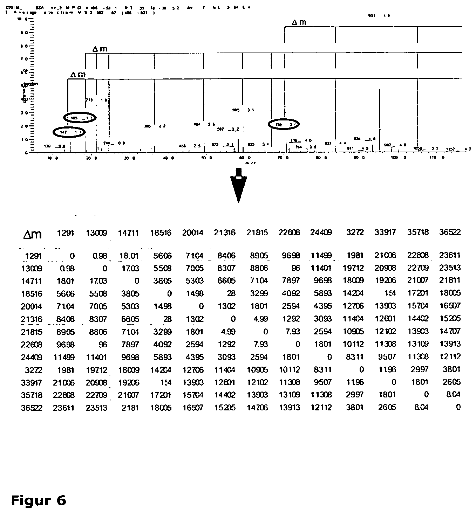

Die extrahierten Massen werden untereinander in Bezug gesetzt (Datenbezüge b₁, b₂, b₃), indem ihre Massendifferenzen (Δm). gebildet und in einer Δm-Matrix dargestellt werden (vgl. Figur 6). Hierbei können die Anzahl der zu vergleichenden Signale, die Berücksichtigung von Optimierungsgrößen (bspw. vorheriges Eliminieren von Duplikaten) o.dgl. variieren.

### Schritt 03: Auswerten der Datenbezüge b1, b2, b3

Von allen Massen der Fragmentpeptide eines Fragmentspektrums und der nichtredundanten Menge an Massendifferenzen der zugehörigen Δm-Matrix wird eine Datenanfrage an die vorab erzeugte Datenbasis 110 gestellt.

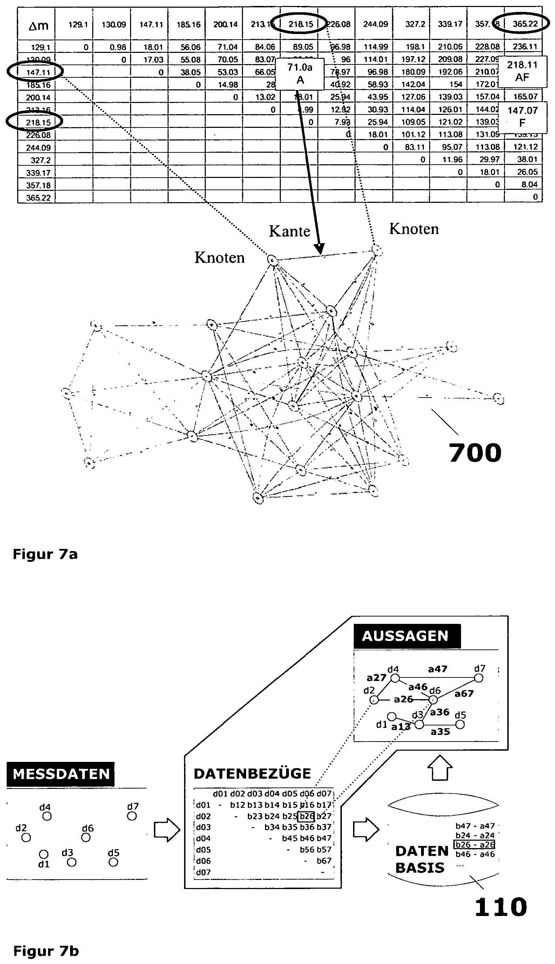

Aus den Ergebnissen der Datenanfrage wird ein ungerichteter Graph 700 erstellt (vgl. Figur 7). Hierbei wird für jede Masse ein Knoten erzeugt. Alle Massendifferenzen, für die ein Eintrag in der Datenbasis 110 existiert, werden als Kanten zwischen den zugehörigen Knoten definiert und mit dem Ergebnis der Anfrage versehen. Knoten ohne Bezüge werden ignoriert. Bei der Auswerten der Datenbezüge b1, b2, b3 können die Anzahl der angefragten Massenabstände pro Fragmentspektrum (Größe der Matrix), die Anzahl der Einträge der angefragten Datenbasis, vorgenommene Optimierungen (bspw. Eliminieren unbrauchbarer Massenabstände (z.B. Anzahl der gefundenen Aminosäure-Entsprechungen sind zu groß, so dass keine verwertbaren Informationen gewonnen werden können)) o.dgl. variieren.

### Schritt 04: Ableiten von Aussagen a₁, a₂, a₃

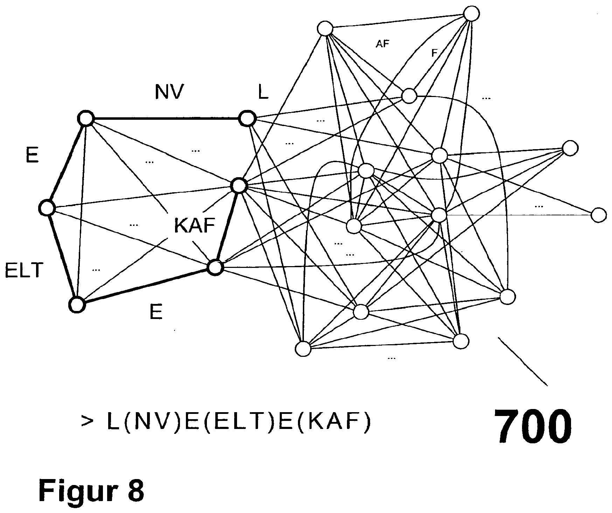

Aussagen a₁, a₂, a₃ werden an das Auffinden von Pfaden durch den in Schritt 03 entstanden Graphen 700 gebunden (vgl. Figur 8). Den Startpunkt bildet in einer beispielhaften Ausführungsform der Knoten mit der niedrigsten Masse. Gesucht werden alle Pfade, die entlang der Kanten zu Knoten jeweils höherer Masse verlaufen, solange bis der Knoten, der die Masse des Peptids repräsentiert, erreicht ist. Die entstehenden Pfade entsprechen möglichen Teilen von Fragmentserien des untersuchten Peptids. (Alternativ kann auch ausgehend vom Knoten mit der höchsten Masse gesucht werden. Der Algorithmus wird dann entsprechend in naheliegender Weise abgewandelt, beispielsweise indem entlang der Kanten zu Knoten jeweils niedrigerer Masse gegangen wird.)

Können dem Startknoten und allen Kanten ausschließlich eindeutige Aminosäureformen zugeordnet werden, so ergibt sich eine eindeutige und vollständige Fragmentserie und folglich die Sequenzabfolge des Peptids. Uneindeutige Kanten zeigen dagegen Sequenzbereiche des Peptids an, die für eine Identifizierung in den folgenden Messungen weiter untersucht werden müssen. Beim Ableiten von Aussagen a₁, a₂, a₃ können die Größe (Anzahl der Knoten) des entstandenen Graphen, der Verknüpfungsgrad und die Eindeutigkeit des entstandenen Graphen, die Vollständigkeit der gefundenen Massenabstände o.dgl. variieren.

### Schritt 05: Abgleichen von Aussage a₁, a₂, a₃ und Zielstellung

Aus den abgeleiteten Aussagen a₁, a₂, a₃ der vorangegangenen Auswertung, d.h. der erreichten Identifizierung der Aminosäuresequenz des untersuchten Peptids durch eine Online De-novo Sequenzierung, wird festgestellt, wie vollständig und eindeutig das gestellte (Teil-)Ziel erreicht ist. Hierbei kann beispielsweise der Grad der Sequenzabdeckung o.dgl. variieren.

### Schritt 06: Zielorientiertes Variieren der Geräteparameter, Messmodus

Aus den bisher abgeleiteten Aussagen a₁, a₂, a₃, der bisherigen Zielabdeckung, sowie der im Messverlauf verfügbaren Peptidionen werden die Geräteparameter p₁, p₂, p₃ und damit der folgende Messverlauf zielgerichtet bestimmt. Hierbei kann beispielsweise die vorhandene Signalstärke (Signalverlauf im Chromatogramm) o.dgl. variieren.

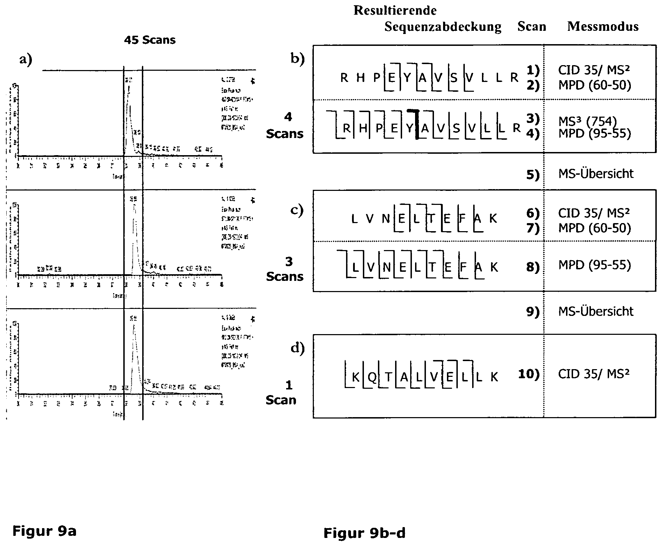

Besonders vorteilhaft wird die Erfindung beispielsweise bei einem zeitkritischen HPLC/MS-Lauf eingesetzt. Während eines HPLC/MS-Messlaufes (Messexperiment) können verschiedene Peptide eines Ausgangsgemisches gleichzeitig in das Massenspektrometer gelangen. Die Konzentration jedes Peptids durchläuft dabei ein, durch das Trennsystem bedingtes Maximum, bevor das Peptid nicht mehr detektiert werden kann (Figur 9a). Durch die Überlagerung der einzelnen Konzentrationsverläufe ist eine sequenzielle Messung der Peptide erschwert. Es muss also eine Prioritätenabfolge, abhängig von der Verfügbarkeit eines Peptids festgelegt werden. Wird der Bedarf an Messungen für jedes Peptid, um es eindeutig zu identifizieren, so gering wie möglich gehalten, so steigt die Anzahl an Peptiden, die insgesamt analysiert werden können.
Anhand eines bereits durchgeführten HPLC/MS Messlaufs wurde jedes Massenspektrum dahingehend analysiert, inwieweit die bereits durchgeführte Fragmentierung des Peptids zur Identifizierung führte. In diesem Fall hätte jede weitere Messung im gleichen Messmodus für die Sequenzierung weiterer Peptide genutzt werden können.
Im Fall einer uneindeutigen Sequenzierung eines Peptids wurden Messungen mit variiertem Messmodus am entsprechenden Peptid herangezogen. Bei einer vollständigen und eindeutigen Sequenzierung bzw. dem Erreichung der maximal möglichen Messungen wurde die Fragmentierung des Peptids beendet und die bis dato extrahierte (Teil-)Sequenz sowie die Erhöhung der Sequenzabdeckung (im Vergleich zu bisherigen Methoden) ausgegeben. Dies ist in den Figuren 9b-c am Beispiel von drei Peptiden dargestellt, die in einem Zeitfenster von etwa 1.5 Min auftreten.

Ein weiteres Beispiel ist die Auswertung eines Fragmentspektrums eines MeCAT-Lu modifizierten Peptids P3. Als Fragmentierungsmethode wurde IRMPD verwendet. Große Teile der Sequenz sind nicht eindeutig (obere Markierung b-Serie, untere Markierung y-Serie).

Nach verändertem Messmodus mit ECD als Fragmentierungsmethode, wurde die Substanz erneut vermessen und das resultierende Massenspektrum ausgewertet. Das Peptid ist nun durch eine fast vollständige c-Serie (obere Markierungen) und einige Fragmente der z-Serie (untere Markierungen) bis auf die Reihenfolge von zwei Aminosäuren am N-Terminus vollständig und eindeutig identifiziert.

Versuche mit den bisherigen, vordefinierten Messverfahren haben gezeigt, dass die entstehenden Massenspektren einen Pool an unnötigen Messungen bedingen. Hierzu zählen bspw. Mengen identischer Messungen (redundante Daten), unnötige Messungen einer, zum aktuellen Zeitpunkt bereits bekannten Aussage sowie eine Menge an unbrauchbaren Messungen. Daraus resultiert, dass Messungen "verschwendet" werden und Teilsequenzen ggf. nur uneindeutig und unvollständig identifiziert werden.

Im Vergleich dazu wurden die entstehenden Massenspektren zunächst offline durch DBnovo im gesetzten Zeitfenster (1s) ausgewertet, um mit Hilfe der resultierenden Aussagen die Sequenzbereiche der Peptide aufzuzeigen, die für eine vollständige Sequenzabdeckung noch benötigt werden.

Durch die Wahl eines zielorientiert veränderten Messmodus aus einer Menge an festgelegten Grundmodi konnte so nachweislich mittels erneuter Fragmentierung des gesamten Peptids (MS²) bzw. eines ausgewählten Fragments (MS3) eine vollständige und eindeutige Sequenzierung erreicht werden.

Es ist somit durch Einsatz der Erfindung möglich, die Identifizierung durch situationsspezifisches Verändern des Messverlaufs zu forcieren und durch Erkennen (des Vorliegens) einer vollständig und eindeutig aufgeklärten Sequenz den Fokus zeitnah auf ein weiteres Peptid zu lenken.

Die Erfindung beschränkt sich in ihrer Ausführungsform nicht auf die vorstehend angegebenen bevorzugten Ausführungsbeispiele. Vielmehr ist eine Anzahl von Varianten denkbar, wobei der Schutzbereich der Erfindung durch die Ansprüche definiert ist.

### Bezugszeichenliste

- 100: Messgerät
- 110: Datenbasis
- 120: Messstrategie (Variationsstrategie)
- 700: Graph

## Patentansprüche

1. Verfahren zur Steuerung eines Messsystems, das mindestens ein Massenspektrometer umfasst, wobei
eine Datenbasis bereitgestellt wird, umfassend Informationen über zumindest einen Teil der zu vermessenden Messobjekte, über zumindest einen Teil der Messdaten (d₁, d₂, d₃), die bei mit dem Messsystem durchgeführten Messexperimenten erfassbar sind, und umfassend Informationen über Beziehungen zwischen zumindest einem Teil der Messdaten (d₁, d₂, d₃);
während des zeitlichen Verlaufs eines Messexperiments
- erfasste Messdaten (d₁, d₂, d₃) automatisch ausgewertet werden, wobei die Auswertung eine Ermittlung von Beziehungen zwischen zumindest einem Teil der erfassten Messdaten (d₁, d₂, d₃) umfasst;
- mindestens eine Anfrage an die Datenbasis erfolgt, um mit Hilfe der ermittelten Beziehungen Informationen über erfasste Messobjekte zu erhalten; und
- in Abhängigkeit der Informationen über erfasste Messobjekte und/oder Messdaten (d₁, d₂, d₃) das Messsystem gesteuert wird; wobei die zu vermessenden Messobjekte Biopolymere und/oder deren Derivate umfassen und wobei bei der Anfrage an die Datenbasis zumindest ein Teil der Massen von Biopolymerfragmenten eines Fragmentspektrums und zumindest ein Teil der Massendifferenzen zwischen den Biopolymerfragmenten als Anfrageparameter dienen,
wobei durch die Anfrage an die Datenbasis mögliche Kandidaten für unaufgeklärte Sequenzteile der Biopolymere ermittelt werden, die bei einer massenspektrometrischen Fragmentierung entstehen,
wobei in Abhängigkeit der ermittelten unaufgeklärten Sequenzteile
- Vorläufer für weitere Fragmentierungen bestimmt,
- Fragmentierungsmethoden gewählt und/oder variiert und/oder
- Fragmentierungsparameter angepasst und/oder variiert werden.

2. Verfahren nach Anspruch 1, wobei
- die Messdaten (d₁, d₂, d₃) Masse-/Ladung-Verhältnisse und/oder die Intensität von Signalen und/oder physikalischen Größen und/oder daraus ableitbare Größen umfassen, die durch das Messsystem ermittelt werden können, wobei die Biopolymere und/oder deren Derivate aus Monomeren aufgebaut sind, die bevorzugt auch modifizierte Monomere umfassen, und eine Untereinheit eines Biopolymers und/oder eines Derivats, welche mehrere Monomere umfasst, ein Polymer bildet, wobei bevorzugt die Biopolymere oder Derivate auch modifizierte Biopolymere bzw. modifizierte Derivate umfassen.

3. Verfahren nach einem der voranstehenden Ansprüche, wobei es sich bei den Biopolymeren oder Derivaten um Proteine, Peptide, DNA, RNA, PNA, LNA, TNA, GNA, Polysaccharide, wie Stärke, Cellulose oder Glykogen, um Lipide, Polyglucosamine, wie Chitin oder Chitosan, um Polyhydroxyalkanoate, Cutin, Suberin oder Lignin oder Kombinationen davon handelt.

4. Verfahren nach einem der vorstehenden Ansprüche, wobei die Datenbasis
- Vorberechnete Einzelergebnisse (e₁, e₂, e₃),
- Informationen über Beziehungen zwischen zumindest einem Teil der vorberechneten Einzelergebnisse (e₁, e₂, e₃),
- Informationen über physikalische und/oder chemische Eigenschaften der Messobjekte und/oder
- Informationen aus vorangegangenen Messungen und/oder Messexperimenten umfasst.

5. Verfahren nach einem der vorstehenden Ansprüche, wobei die Datenbasis
- Massendifferenzen zwischen Monomeren und/oder Polymeren der Biopolymere und/oder
- Intensitätsdifferenzen umfasst, wobei bevorzugt die Datenbasis Massendifferenzen zwischen Polymeren mit Abständen von einem oder mehreren Monomeren umfasst.

6. Verfahren nach einem der voranstehenden Ansprüche, wobei durch die Anfrage an die Datenbasis aufgeklärte Sequenzteile der Biopolymere ermittelt werden, die bei einer massenspektrometrischen Fragmentierung entstehen, wobei bevorzugt ermittelte aufgeklärte Sequenzteile mit in anderen Messungen oder Messexperimenten gewonnenen Sequenzteilen kombiniert werden zur Ermittlung der Vollständigkeit des Sequenzierungsgrades, und wobei besonders bevorzugt aus ermittelten aufgeklärten Sequenzteilen die Zugehörigkeit zu Biopolymergruppen abgeleitet wird.

7. Verfahren nach einem der voranstehenden Ansprüche, wobei aus den Ergebnissen der Anfrage zwei Massen von Biopolymerfragmenten einander zugeordnet und entsprechende Zuordnungsinformationen erzeugt werden, wenn in der Datenbasis mindestens ein Eintrag gefunden wird, welcher der Massendifferenz zwischen diesen beiden Massen entspricht, wobei bevorzugt die Zuordnungsinformationen außerdem Angaben über ein oder mehrere Monomere umfasst, welche die Massendifferenz zwischen den beiden Massen repräsentieren.

8. Verfahren nach einem der voranstehenden Ansprüche, wobei bei der Ermittlung von mögliche Kandidaten für unaufgeklärte Sequenzteile der Biopolymere Verfahren der Graphentheorie, Verfahren der künstlichen Intelligenz, wie Fuzzy-Logik, Verfahren zur Verwendung von Neuralen Netzwerken und/oder genetischen Algorithmen und/oder Evolutionsstrategien und/oder Datenbasisanfragen eingesetzt werden.

9. Verfahren nach einem der voranstehenden Ansprüche, wobei durch Auswertung der einander zugeordneten Massen oder Zuordnungsinformationen mögliche Kandidaten für unaufgeklärte Sequenzteile der Biopolymere ermittelt werden, indem zwischen Massen, ausgehend von einer Masse, mindestens eine Verknüpfungskette bestimmt wird, welche Elemente, denen jeweils eine Masse zugeordnet ist, und Verknüpfungen, denen jeweils Angaben über ein oder mehrere Monomere zugeordnet sind, welche die Massendifferenz zwischen den beiden verknüpften Massen repräsentieren, umfasst, wobei die mindestens eine Verknüpfungskette stets jeweils in Richtung auf- oder absteigender Massen verläuft, und wobei mögliche Kandidaten für unaufgeklärte Sequenzteile der Biopolymere durch Verknüpfungen angezeigt werden, denen mindestens zwei Monomere zugeordnet sind.

10. Verfahren nach Anspruch 6, wobei als Kriterium für das Vorhandensein unaufgeklärter Sequenzteile folgende Situationen dienen:
- es existieren Verknüpfungen, denen mindestens zwei Monomere zugeordnet sind,
- die niedrigste Masse entspricht nicht der Masse eines einzelnen Monomers oder
- es existieren Verknüpfungen, der höchste Masse zur Masse des unfragmentierten Biopolymers, denen mindestens zwei Monomere zugeordnet sind und/oder die vollständige Aufklärung einer Sequenz durch eine Verknüpfungskette angezeigt wird, die ausschließlich aus Monomeren besteht.

11. Verfahren nach einem der voranstehenden Ansprüche, wobei das Messsystem derart gesteuert wird, dass in Abhängigkeit der Informationen über erfasste Messobjekte aus einem ersten Messvorgang während des Messexperiments der direkt auf den ersten Messvorgang folgende oder ein späterer Messvorgang parametrisiert wird, wobei bevorzugt die Parameter für einen Messvorgang während des Messexperiments in Abhängigkeit einer Aufgabenliste gewählt werden.

12. Verfahren nach einem der voranstehenden Ansprüche, wobei in Abhängigkeit der ermittelten Kandidaten für unaufgeklärte Sequenzteile der Biopolymere Geräteparameter (p₁, p₂, p₃), des Messsystems variiert werden, wobei bevorzugt die Variation der Geräteparameter (p₁, p₂, p₃) in Abhängigkeit davon erfolgt, ob das das unaufgeklärte Sequenzteil repräsentierende Signal noch vorhanden ist.

13. Verfahren nach Anspruch 12, wobei die Geräteparameter (p₁, p₂, p₃) variiert werden, indem die Geräteparameter der nächsten Messung aufgrund der aktuellen Messung festgelegt werden oder dass nach einer Auswertung der Messdaten (d₁, d₂, d₃) eine Messung mit veränderten Geräteparametern in einer Messliste eingetragen wird, die abgearbeitet wird.

14. Verfahren nach einem der voranstehenden Ansprüche, wobei die Ermittlung unaufgeklärter Sequenzteile und die Steuerung im Wesentlichen in Echtzeit während des Messvorgangs erfolgt.

15. Verfahren nach einem der voranstehenden Ansprüche, wobei das Messsystem ferner einen HPLC-Chromatographen umfasst und wobei eine Prioritätenabfolge im zeitlichen Verlauf des Messexperiments für einzelne Peptide der Biopolymere und/oder deren Derivate in Abhängigkeit von der Verfügbarkeit der Peptide festgelegt wird.

16. Verfahren nach Anspruch 15, wobei die Verfügbarkeit eines Peptids anhand des Konzentrationsverlaufs des Peptids im Chromatographen ermittelt wird.

17. Verfahren nach Anspruch 15 oder 16, wobei anhand eines bereits durchgeführten Massenspektrometrie/Chromatographie-Messexperiments ein als erfasste Messdaten ermitteltes Massenspektrum dahingehend analysiert wird, inwieweit eine bereits durchgeführte Fragmentierung eines Peptids zur Identifizierung führt.

18. Verfahren nach Anspruch 17, wobei anhand der Analyse des ermittelten Massenspektrums entschieden wird, ob eine weitere Messung im gleichen Messmodus an weiteren Peptiden oder mit variiertem Messmodus am gleichen Peptid durchgeführt wird.

19. Anordnung mit mindestens einem Chip und/oder Prozessor, wobei die Anordnung derart eingerichtet ist, dass ein Verfahren gemäß einem der Ansprüche 1 bis 18 ausführbar ist, wobei die Anordnung weiter mindestens einen Massenspektrometer und eventuell ein NMR-Gerät und/oder weitere Geräte, insbesondere einen Laser, umfasst, die in Abhängigkeit der Informationen über erfasste Messobjekte gesteuert werden.

20. Computerprogramm, das es einer Datenverarbeitungseinrichtung ermöglicht, nachdem es in Speichermittel der Datenverarbeitungseinrichtung geladen worden ist, während des zeitlichen Verlaufs eines Messexperiments ein Verfahren gemäß einem der Ansprüche 1 bis 18 durchzuführen.

21. Computerlesbares Speichermedium, auf dem ein Programm gespeichert ist, das es einer Datenverarbeitungseinrichtung ermöglicht, nachdem es in Speichermittel der Datenverarbeitungseinrichtung geladen worden ist, ein Verfahren gemäß einem der Ansprüche 1 bis 18 durchzuführen.

22. Verfahren, bei dem ein Computerprogramm nach Anspruch 20 aus einem elektronischen Datennetz, wie beispielsweise aus dem Internet, auf eine an das Datennetz angeschlossene Datenverarbeitungseinrichtung heruntergeladen wird.

## Claims

1. Method for controlling a measuring system comprising at least one mass spectrometer,
wherein
a database is provided, comprising information regarding at least a part of the measurement objects to be measured, and regarding at least a part of the measurement data (d₁, d₂, d₃) that can be recorded during measurement experiments carried out by the measuring system, and comprising information regarding relationships between at least a part of the measurement data (d₁, d₂, d₃);
during the course of a measurement experiment over time,
- recorded measurement data (d₁, d₂, d₃) are automatically evaluated, wherein the evaluation comprises a determination of relationships between at least a part of the recorded measurement data (d₁, d₂, d₃);
- at least one query is made to the database in order to obtain information regarding recorded measurement objects using the determined relationships; and
- the measuring system is controlled according to the information regarding recorded measurement objects and/or measurement data (d₁, d₂, d₃); wherein the measurement objects to be measured comprise biopolymers and/or the derivatives thereof and wherein at least a part of the masses of biopolymer fragments of a fragment spectrum and at least a part of the mass differences between the biopolymer fragments are used as query parameters when the query is made to the database,
wherein, by means of the query to the database, possible candidates for unelucidated sequence portions of the biopolymers are determined that result from mass-spectrometric fragmentation,
wherein, depending on the determined unelucidated sequence portions,
- precursors for further fragmentation are identified,
- fragmentation methods are selected and/or varied and/or
- fragmentation parameters are adjusted and/or varied.

2. Method according to claim 1, wherein
- the measurement data (d₁, d₂, d₃) comprise mass/charge ratios and/or the intensity of signals and/or physical quantities and/or quantities derivable therefrom, which can be determined by the measuring system, wherein the biopolymers and/or the derivatives thereof are built up from monomers, which preferably also include modified monomers, and a subunit of a biopolymer and/or a derivative which comprises a plurality of monomers forms a polymer, wherein the biopolymers or derivatives preferably also comprise modified biopolymers or modified derivatives.

3. Method according to any of the preceding claims, wherein the biopolymers or derivatives are proteins, peptides, DNA, RNA, PNA, LNA, TNA, GNA, polysaccharides such as starch, cellulose or glycogen, lipids, polyglucosamines such as chitin or chitosan, polyhydroxyalkanoates, cutin, suberin or lignin or combinations thereof.

4. Method according to any of the preceding claims, wherein the database comprises
- pre-calculated individual results (e₁, e₂, e₃),
- information regarding relationships between at least a part of the precalculated individual results (e₁, e₂, e₃),
- information regarding physical and/or chemical properties of the measurement objects and/or
- information from previous measurements and/or measurement experiments.

5. Method according to any of the preceding claims, wherein the database comprises
- mass differences between monomers and/or polymers of the biopolymers and/or
- intensity differences, wherein the database preferably comprises mass differences between polymers at intervals one or more monomers.

6. Method according to any of the preceding claims, wherein, by means of the query to the database, elucidated sequence portions of the biopolymers are determined that result from mass-spectrometric fragmentation, wherein determined elucidated sequence portions are preferably combined with sequence portions obtained in other measurements or measurement experiments, to determine the completeness of the degree of sequencing, and wherein membership to biopolymer groups is particularly preferably derived from determined elucidated sequence portions.

7. Method according to any of the preceding claims, wherein, from the results of the query, two masses of biopolymer fragments are assigned to one another and corresponding assignment information is generated if, in the database, at least one entry is found which corresponds to the mass difference between these two masses, wherein the assignment information preferably also comprises details regarding one or more monomers that represent the mass difference between the two masses.

8. Method according to any of the preceding claims, wherein methods of graph theory, methods of artificial intelligence such as fuzzy logic, methods for using neural networks and/or genetic algorithms and/or evolution strategies and/or database queries are used in the determination of possible candidates for unelucidated sequence portions of the biopolymers.

9. Method according to any of the preceding claims, wherein, by evaluating the mutually assigned masses or assignment information, possible candidates for unelucidated sequence portions of the biopolymers are determined by at least one linkage chain being determined between masses, starting from one mass, which chain comprises elements to each of which a mass is assigned, and linkages to each of which details regarding one or more monomers that represent the mass difference between the two linked masses is assigned, wherein the at least one linkage chain always extends in the direction of ascending or descending masses, and where possible candidates for unelucidated sequence portions of the biopolymers are indicated by linkages to which at least two monomers are assigned.

10. Method according to claim 6, wherein the following situations are used as the criterion for the presence of unelucidated sequence portions:
- there are linkages to which at least two monomers are assigned,
- the lowest mass does not correspond to the mass of a single monomer or
- there are linkages, of the highest mass to the mass of the unfragmented biopolymer, to which at least two monomers are assigned and/or the complete elucidation of a sequence is indicated by a linkage chain that consists exclusively of monomers.

11. Method according to any one of the preceding claims, wherein the measuring system is controlled such that, depending on the information regarding recorded measurement objects from a first measuring process during the measurement experiment, the measuring process directly following the first measuring process or a later measuring process is parameterized, wherein the parameters for a measuring process during the measurement experiment can preferably be selected depending pn a task list.

12. Method according to any one of the preceding claims, wherein, according to the determined candidates for unelucidated sequence portions of the biopolymers, device parameters (p₁, p₂, p₃) of the measuring system are varied, wherein the device parameters (p₁, p₂, p₃) are preferably varied according to whether the signal representing the unelucidated sequence portion is still present.

13. Method according to claim 12, wherein the device parameters (p₁, p₂, p₃) are varied by the device parameters of the next measurement being set on the basis of the current measurement or by, after an evaluation of the measurement data (d₁, d₂, d₃), a measurement having changed device parameters being entered in a measurement list that is processed.

14. Method according to any one of the preceding claims, wherein the determination of unelucidated sequence portions and the control take place substantially in real time during the measuring process.

15. Method according to any one of the preceding claims, wherein the measuring system further comprises an HPLC chromatograph and wherein a sequence of priorities in the course of the measurement experiment over time for individual peptides of the biopolymers and/or the derivatives thereof is set depending on the availability of the peptides.

16. Method according to claim 15, wherein the availability of a peptide is determined on the basis of the concentration course of the peptide in the chromatograph.

17. Method according to either claim 15 or claim 16, wherein, on the basis of a mass spectrometry/chromatography measurement experiment that has already been carried out, a mass spectrum determined as recorded measurement data is analyzed in terms of the extent to which fragmentation of a peptide that has already been carried out leads to identification.

18. Method according to claim 17, wherein, on the basis of the analysis of the determined mass spectrum, it is decided whether a further measurement is carried out in the same measurement mode on further peptides or with a different measurement mode on the same peptide.

19. Arrangement comprising at least one chip and/or processor, wherein the arrangement is set up such that a method according to any one of claims 1 to 18 can be carried out, wherein the arrangement further comprises at least one mass spectrometer and potentially an NMR device and/or further devices, in particular a laser, which are controlled depending on the information regarding recorded measurement objects.

20. Computer program which allows a data processing device, after it has been loaded into storage means of the data processing device, to carry out a method according to any one of claims 1 to 18 during the course of a measurement experiment over time.

21. Computer-readable storage medium on which a program is stored which allows a data processing device, after it has been loaded into storage means of the data processing device, to carry out a method according to any one of claims 1 to 18.

22. Method in which a computer program according to claim 20 is downloaded from an electronic data network, for example from the Internet, to a data processing device connected to the data network.

## Revendications

1. Procédé permettant de commander un système de mesure comprenant au moins un spectromètre de masse, dans lequel
une base de données est fournie, comprenant des informations sur au moins une partie des objets de mesure à mesurer, sur au moins une partie des données de mesure (d₁, d₂, d₃) qui peuvent être enregistrées par les expériences de mesure réalisées au moyen du système de mesure, et comprenant des informations sur des relations entre au moins une partie des données de mesure (d₁, d₂, d₃) ;
au cours de l'évolution dans le temps d'une expérience de mesure
- des données de mesure (d₁, d₂, d₃) enregistrées sont automatiquement évaluées, l'évaluation comprenant une détermination des relations entre au moins une partie des données de mesure (d₁, d₂, d₃) enregistrées ;
- au moins une requête est envoyée à la base de données pour obtenir des informations sur les objets de mesure enregistrés à l'aide des relations déterminées ; et
- le système de mesure est commandé en fonction des informations sur des objets de mesure et/ou des données mesurées (d₁, d₂, d₃) enregistrés ; les objets de mesure à mesurer comprenant des biopolymères et/ou leurs dérivés et au moins une partie des masses de fragments de biopolymère d'un spectre de fragments et au moins une partie des différences de masse entre les fragments de biopolymère servant de paramètres de requête lorsque la requête est envoyée à la base de données,
les candidats possibles pour les parties de séquence inexpliquées des biopolymères, qui surviennent suite à une fragmentation spectrométrique de masse, étant déterminés par la requête à la base de données,
en fonction des parties de séquence inexpliquées déterminées,
- un précurseur pour des fragmentations supplémentaires étant défini
- des méthodes de fragmentation étant choisies et/ou variées et/ou
- des paramètres de fragmentation étant ajustés et/ou variés.

2. Procédé selon la revendication 1, dans lequel
- les données de mesure (d₁, d₂, d₃) comprennent des rapports masse/charge et/ou l'intensité de signaux et/ou des grandeurs physiques et/ou des quantités qui peuvent en être dérivées, lesquels peuvent être déterminés au moyen du système de mesure, les biopolymères et/ou leurs dérivés étant constitués de monomères, qui comprennent de préférence également des monomères modifiés, et une sous-unité d'un biopolymère et/ou d'un dérivé, laquelle comprend plusieurs monomères, formant un polymère, les biopolymères ou dérivés comprenant de préférence également des biopolymères modifiés ou des dérivés modifiés.

3. Procédé selon l'une des revendications précédentes, dans lequel les biopolymères ou dérivés sont des protéines, des peptides, de l'ADN, de l'ARN, du PNA, du LNA, du TNA, du GNA, des polysaccharides tels que l'amidon, la cellulose ou le glycogène, des lipides, des polyglucosamines telles que la chitine ou le chitosane, des polyhydroxyalcanoates, de la cutine, de la subérine ou de la lignine ou leurs associations.

4. Procédé selon l'une des revendications précédentes, dans lequel la base de données comprend
- des résultats individuels précalculés (e₁, e₂, e₃),
- des informations sur des relations entre au moins une partie des résultats individuels précalculés (e₁, e₂, e₃),
- des informations sur des propriétés physiques et/ou chimiques des objets de mesure et/ou
- des informations provenant de mesures et/ou d'expériences de mesure précédentes.

5. Procédé selon l'une des revendications précédentes, dans lequel la base de données comprend
- des différences de masse entre des monomères et/ou des polymères des biopolymères et/ou
- des différences d'intensité, la base de données comprenant de préférence des différences de masse entre des polymères avec des espacements d'un ou plusieurs monomères.

6. Procédé selon l'une des revendications précédentes, dans lequel des parties de séquence expliquées des biopolymères, qui résultent d'une fragmentation spectrométrique de masse, sont déterminées par la requête à la base de données, des parties de séquence expliquées déterminées étant de préférence associées avec des parties de séquence obtenues dans d'autres mesures ou expériences de mesure afin de déterminer l'intégralité du degré de séquençage, et l'appartenance à des groupes de biopolymères étant dérivée de manière particulièrement préférée à partir de parties de séquence expliquées déterminées.

7. Procédé selon l'une des revendications précédentes, dans lequel deux masses de fragments de biopolymère sont attribuées l'une à l'autre à partir des résultats de la requête et des informations d'attribution correspondantes sont générées si au moins une entrée est trouvée dans la base de données, à laquelle correspond la différence de masse entre ces deux masses, les informations d'attribution comprenant de préférence également des indications concernant un ou plusieurs monomères, qui représentent la différence de masse entre les deux masses.

8. Procédé selon l'une des revendications précédentes, dans lequel, lors de la détermination de candidats possibles pour des parties de séquence inexpliquées des biopolymères, des procédés de théorie des graphes, des procédés d'intelligence artificielle, telles que la logique floue, des procédés d'utilisation de réseaux neuronaux et/ou d'algorithmes génétiques et/ou de stratégies d'évolution et/ou de demandes de base de données sont utilisés.

9. Procédé selon l'une des revendications précédentes, dans lequel les candidats possibles pour des parties de séquence inexpliquées des biopolymères sont déterminés en évaluant les masses attribuées l'une à l'autre ou les informations d'attribution, en définissant au moins une chaîne de liaison entre les masses, à partir d'une masse, laquelle comprend des éléments auxquels est attribuée une masse respective, et des liaisons auxquelles sont attribuées des indications respectives concernant un ou plusieurs monomères qui représentent la différence de masse entre les deux masses liées, l'au moins une chaîne de liaison s'étendant toujours respectivement dans le sens des masses ascendantes ou descendantes, et des candidats possibles pour des parties de séquence inexpliquées des biopolymères étant indiqués par des liaisons auxquelles au moins deux monomères sont attribués.

10. Procédé selon la revendication 6, dans lequel les situations suivantes sont utilisées comme critères pour la présence de parties de séquence inexpliquées :
- il existe des liaisons auxquelles au moins deux monomères sont attribués,
- la masse la plus faible ne correspond pas à la masse d'un seul monomère ou
- il existe des liaisons, de la masse la plus élevée à la masse du biopolymère non fragmenté, auxquelles au moins deux monomères sont attribués et/ou l'explication complète d'une séquence est indiquée par une chaîne de liaison constituée exclusivement de monomères.

11. Procédé selon l'une des revendications précédentes, dans lequel le système de mesure est commandé de telle sorte que le processus de mesure suivant directement le premier processus de mesure ou un processus de mesure ultérieur est paramétré en fonction des informations sur des objets de mesure enregistrés provenant d'un premier processus de mesure pendant l'expérience de mesure, les paramètres pour un processus de mesure pouvant être sélectionnés de préférence pendant l'expérience de mesure en fonction d'une liste de tâches.

12. Procédé selon l'une des revendications précédentes, dans lequel, en fonction des candidats déterminés pour des parties de séquence inexpliquées de biopolymères, des paramètres d'appareils (p₁, p₂, p₃) du système de mesure sont variés, la variation des paramètres d'appareils (p₁, p₂, p₃) ayant lieu de préférence si le signal représentant la partie de séquence inexpliquée est encore présent.

13. Procédé selon la revendication 12, dans lequel les paramètres d'appareils (p₁, p₂, p₃) sont variés en établissant les paramètres d'appareils de la prochaine mesure sur la base de la mesure actuelle ou en ce que, après une évaluation des données de mesure (d₁, d₂, d₃), une mesure au moyen de paramètres d'appareils modifiés est entrée dans une liste de mesure, laquelle est traitée.

14. Procédé selon l'une des revendications précédentes, dans lequel la détermination de parties de séquence inexpliquées et la commande ont lieu sensiblement en temps réel pendant le processus de mesure.

15. Procédé selon l'une des revendications précédentes, dans lequel le système de mesure comprend en outre un chromatographe HPLC et dans lequel une séquence de priorités au cours de l'évolution dans le temps de l'expérience de mesure est établie pour des peptides individuels des biopolymères et/ou de leurs dérivés en fonction de la disponibilité des peptides.

16. Procédé selon la revendication 15, dans lequel la disponibilité d'un peptide est déterminée sur la base de l'évolution de la concentration du peptide dans le chromatographe.

17. Procédé selon la revendication 15 ou 16, dans lequel, sur la base d'une expérience de mesure par spectrométrie de masse/chromatographie qui a déjà été réalisée, un spectre de masse déterminé comme données de mesure enregistrées est analysé afin de déterminer dans quelle mesure une fragmentation d'un peptide qui a déjà été réalisée conduit à l'identification.

18. Procédé selon la revendication 17, dans lequel, sur la base de l'analyse du spectre de masse déterminé, il est décidé si une autre mesure est réalisée dans le même mode de mesure sur d'autres peptides ou avec un mode de mesure varié sur le même peptide.

19. Agencement comportant au moins une puce et/ou un processeur, dans lequel l'agencement est configuré de telle sorte qu'un procédé selon l'une des revendications 1 à 18 peut être mis en œuvre, l'agencement comprenant en outre au moins un spectromètre de masse et éventuellement un appareil RMN et/ou d'autres appareils, en particulier un laser, qui sont commandés en fonction des informations sur les objets de mesure enregistrés.

20. Programme informatique permettant à un dispositif de traitement de données, après son chargement dans des moyens de stockage du dispositif de traitement de données, de réaliser un procédé selon l'une des revendications 1 à 18 au cours de l'évolution dans le temps d'une expérience de mesure.

21. Support de stockage lisible par ordinateur sur lequel est stocké un programme qui permet à un dispositif de traitement de données, après son chargement dans des moyens de stockage du dispositif de traitement de données, d'effectuer un procédé selon l'une des revendications 1 à 18.

22. Procédé dans lequel un programme informatique selon la revendication 20 est téléchargé depuis un réseau de données électronique, par exemple depuis Internet, vers un dispositif de traitement de données connecté au réseau de données.
